# EUROPEAN PATENT SPECIFICATION

(11) **EP 0 948 511 B1**
(45) Date of publication and mention of the grant of the patent: **18.10.2006**
(21) Application number: 97953464.1
(22) Date of filing: 19.12.1997
(51) Int. Cl.: C07H 21/00, C07H 19/04, C12N 9/00, C07K 14/08, C12N 15/85, C12N 15/86, C12Q 1/70

(54) **CHEMICAL SYNTHESIS OF NUCLEOSIDES ANALOGS AND THEIR INCORPORATION INTO POLYNUCLEOTIDES**
SYNTHESE VON NUKLEOSIDEN UND POLYNUKLEOTIDEN
SYNTHESE CHIMIQUE D'ANALOGUES DE NUCLEOSIDES ET LEUR INCORPORATION DANS DES POLYNUCLEOTIDES

(30) Priority: 24.12.1996 US 34444 P; 13.02.1997 US 37998 P; 31.03.1997 US 42464 P
(43) Date of publication of application: 13.10.1999
(73) Proprietor: Sirna Therapeutics, Inc., Boulder, CO 80301 (US)
(72) Inventor: KARPEISKY, Alexander, Lafayette, CO 80026 (US); BEIGELMAN, Leonid, Longmont, CO 80503 (US); MATULIC-ADAMIC, Jasenka, Boulder, CO 80303 (US)
(74) Representative: Nobbe, Matthias
(86) International application number: PCT/US1997/023936
(87) International publication number: WO 1998/028317

(56) References cited:
- EP-A- 0 227 844
- WO-A-93/12130
- WO-A-95/01363
- WO-A-95/11304
- WO-A-95/11910
- WO-A-96/18736
- M.PERBOST ET AL.: "Synthesis of 5'-O-Amino-2'-deoxypyrimidine and Purine Nucleosides : Building-Blocks for Antisense Oligonucleotides." JOURNAL OF ORGANIC CHEMISTRY, vol. 60, no. 16, 11 August 1995, pages 5150-5156, XP002062165 cited in the application
- K.BURGESS ET AL.: "Synthesis of an Oxyamide Linked Nucleotide Dimer and Incorporation into Antisense Oligonucleotide Sequences." JOURNAL OF THE CHEMICAL SOCIETY CHEMICAL COMMUNICATIONS, 21 April 1994, pages 915-916, XP002062166 cited in the application

## Description

### Background of the Invention

This invention relates to novel nucleoside analogs, chemical synthesis of these analogs, and their incorporation into polynucleotides

The following is a brief description of nucleoside and chemical modification of oligonucleotides. This summary is not meant to be complete but is provided only for understanding of the invention that follows. This summary is not an admission that all of the work described below is prior art to the claimed invention.

Nucleoside modifications of bases and sugars, have been discovered in a variety of naturally occurring RNA (*e.g*., tRNA, mRNA, rRNA; reviewed by Hall, 1971 *The Modified Nucleosides in Nucleic Acids,* Columbia University Press, New York; Limbach *et al.,* 1994 *Nucleic Acids Res.* 22, 2183). In an attempt to understand the biological significance, structural and thermodynamic properties, and nuclease resistance of these nucleoside modifications in nucleic acids, several investigators have chemically synthesized nucleosides, nucleotides and phosphoramidites with various base and sugar modifications and incorporated them into oligonucleotides.

Uhlmann and Peyman, 1990, *Chem. Reviews* 90, 543, review the use of nucleoside modifications to stabilize antisense oligonucleotides.

Usman *et al.,* International PCT Publication Nos. *WO*/*93*/*15187;* and *WO 95*/*13378;* describe the use of sugar, base and backbone modifications to enhance the nuclease stability of enzymatic nucleic acid molecules.

Eckstein *et al.,* International PCT Publication No. *WO 92*/*07065* describe the use of sugar, base and backbone modifications to enhance the nuclease stability of enzymatic nucleic acid molecules.

Grasby *et al.,* 1994, *Proc. Indian Acad. Sci.,* 106, 1003, review the "applications of synthetic oligoribonucleotide analogues in studies of RNA structure and function".

Eaton and Pieken, 1995, *Annu. Rev. Biochem.,* 64, 837, review sugar, base and backbone modifications that enhance the nuclease stability of RNA molecules

Hildbrand and Leumann, 1996, *Agnew. Chem. Int. Ed. Engl.,* 35, 1968, describe a method for the synthesis of a 2-aminopyridine nucleoside derivative.

Mitsunobu, 1981, *Synthesis,* 1, 1-28, described a process for the conversion of alcohol (ROH) to aminooxy alcohol (RONH₂).

The process descibed by Mitsunobu *(supra)* has been been applied in the conversion of sugars and disaccharides (Grochowski *et al.,* 1976, 50, C15; *Synthesis* 1976, 682; *J. Bull. Pol. Acad Sci. Chem. Commun.,* 1987, 35, 255; Tronchet *et al.,* 1982, *Helv. Chim. Acta.,* 65,1404; *Carbohydr. Res.,* 1990,204).

The process descibed by Mitsunobu *(supra)* has also been applied in the synthesis of 3'-O-NH₂ nudeosides and 5'-O-NH₂ nucleosides (Nielsen, 1995, *Annu. Rev. Biomol. Struc.,* 24, 167; Burgess *et al.,* 1994, *J. Chem. Soc. Chem. Commun.,* 915; Kondo *et al.,* 1985, *Am. Chem. Soc. Symp. Ser.,* 16, 93; Vasseur *et al.,* 1992, *J. Am. Chem. Soc*., 114, 4006; Tronchet *et al.,* 1994, 13, 2071; Perbost *et al.,* 1995, *J*. *Org. Chem.,* 60, 5150).

The information disclosed in the references cited above are distinct from the presently claimed invention since they do not disclose and/or contemplate the processes for the synthesis of the methoxy nucleosides as claimed in the instant invention.

This invention relates to a nucleoside or a nucleotide comprising a nucleic acid sugar portion, wherein the 2' position of said sugar has the formula: 2'-O-NHR1, wherein R1 is independently H, aminoacyl group, peptidyl group, biotinyl group, cholesteryl group, lipoic acid residue, retinoic acid residue, folic acid residue, ascorbic acid residue, nicotinic acid residue, 6-aminopenicillanic acid residue, 7-aminocephalosporanic acid residue, alkyl, alkenyl, alkynyl, aryl, alkylaryl, carbocyclic aryl, heterocyclic aryl, amide or ester. The invention also relates to a nucleoside or a nucleotide comprising a nucleic acid sugar portion, wherein the 2' position of said sugar has the formula: 2'-*O*-N=R3, wherein R3 is independently pyridoxal residue, pyridoxal-5-phosphate residue, 13-cis-retinal residue, 9-cis-retinal residue, alkyl, alkenyl, alkynyl, alkylaryl, carbocyclic alkylaryl, or heterocyclic alkylaryl

This invention further relates to novel nucleosides or nucleotides having the Formula I 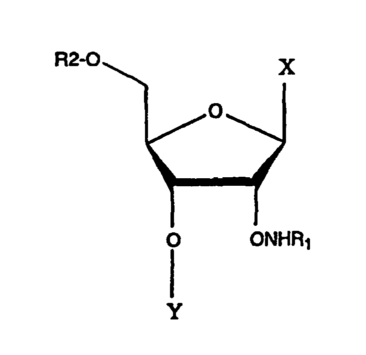 wherein, R1 is independently H, aminoacyl group, peptidyl group, biotinyl group, cholesteryl group, lipoic acid residue, retinoic acid residue, folic acid residue, ascorbic acid residue, nicotinic acid residue, 6-aminopenicillanic acid residue, 7-aminocephalosporanic acid residue, alkyl, alkenyl, alkynyl, aryl, alkylaryl, carbocyclic aryl, heterocyclic aryl, amide or ester, X is independently a nucleotide base or its analog ; Y is independently a phosphorus-containing group; and R2 is independently blocking group or a phosphorus-containing group.

In one embodiment the invention features the above novel nucleosides or nucleotides having the Formula II: 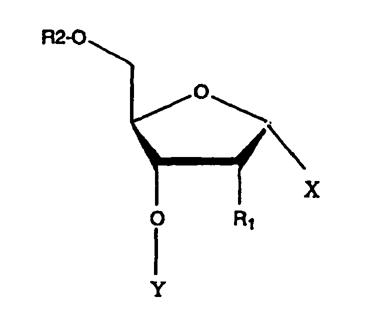 wherein, R1 is defined as above ; X is independently a nucleotide base selected from a group consisting of 2-fluoropyridine-3-yl, 2-bromopyridine-5-yl, pyridin-2-one-5-yl, and 2-aminopyridine-5-yl; Y is independently a phosphorus-containing group; and R2 is independently a blocking group phosphorus-containing group.

In a referred embodiment the invention features a nucleoside or nucleotide having the Formula III: 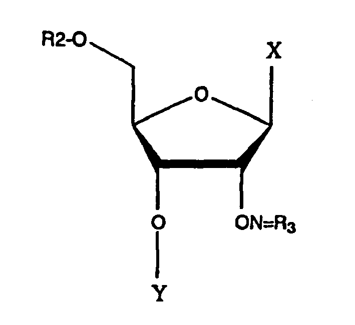 wherein, R3 is independently pyridoxal residue, pyridoxal-5-phosphate residue, 13-cis-retinal residue, 9-cis-retinal residue, alkyl, alkenyl , alkynyl, alkylaryl, carbocyclic alkylaryl, or heterocyclic alkylaryl; X is independently a nucleotide base or its analog ; Y is independently a phosphorus-containing group; and R2 is independently blocking group or a phosphorus-containing group.

A "blocking group" is a group which is able to be removed after polynucleotide synthesis and/or which is compatible with solid phase polynucleotide synthesis.

A "phosphorus containing group" can include phosphorus in forms such as dithioates, phosphoramidites and/or as part of an oligonucleotide.

In a preferred embodiment, the invention features a process for the synthesis of the nucleosides or nucleotides of the invention.

By "alkyl" group is meant a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxy, cyano, alkoxy, NO₂ or N(CH₃)₂, amino, or SH.

The term "alkenyl" group refers to unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, NO₂, halogen, N(CH₃)₂, amino, or SH.

The term "alkynyl" refers to an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, NO₂ or N(CH₃)₂, amino or SH.

An "aryl" group refers to an aromatic group which has at least one ring having a conjugated n electron system and includes carbocyclic aryl, heterocyclic aryl and biaryl groups, all of which may be optionally substituted. The preferred substituent(s) on aryl groups are halogen, trihalomethyl, hydroxyl, SH, cyano, alkoxy, alkyl, alkenyl, alkynyl, and amino groups.

An "alkylaryl" group refers to an alkyl group (as described above) covalently joined to an aryl group (as described above).

"Carbocyclic aryl" groups are groups wherein the ring atoms on the aromatic ring are all carbon atoms. The carbon atoms are optionally substituted.

"Heterocyclic aryl" groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, pyrrolo, pyrimidyl, pyrazinyl, imidazolyl and the like, all optionally substituted.

An "amide" refers to an -C(O)-NH-R, where R is either alkyl, aryl, alkylaryl or hydrogen.

An "ester" refers to an -C(O)-OR', where R is either alkyl, aryl, alkylaryl or hydrogen.

In yet another preferred embodiment, the invention features the incorporation of one or more of the novel nucleosides, nucleotides, or combinations thereof, into polynucleotides. These novel nucleosides and nucleotides can be incorporated into polynucleotides enzymatically. For example by using bacteriophage T7 RNA polymerase, these novel nucleosides or nucleotides can be incorporated into RNA at one or more positions (Milligan *et al.,* 1989, *Methods Enzymol.,* 180, 51). Alternatively, the novel nucleosides or nucleotides can be incorporated into polynucleotides using solid phase synthesis (Brown and Brown, 1991, in *Oligonucleotides and Analogues: A Practical Approach,* p. 1, ed. F. Eckstein, Oxford University Press, New York; Wincott *et al.,* 1995, *Nucleic Acids Res*., 23, 2677; Beaucage & Caruthers, 1996, in *Bioorganic Chemistry: Nucleic Acids*, p 36, ed. S. M. Hecht, Oxford University Press, New York).

The novel nucleosides of the invention can be used for chemical synthesis of nucleotides, nucleotide-tri-phosphates and/or phosphoramidites as a building block for selective incorporation into oligonucleotides. These oligonucleotides can be used as an enzymatic nucleic acid molecule. The oligonucleotides can also be used as probes or primers for synthesis and/or sequencing of RNA or DNA.

The novel nucleosides, nucleotides, or combinations thereof, can also be independently or in combination used as an antiviral, anticancer or an antitumor agent. These compounds can also be independently or in combination used with other antiviral, anticancer or an antitumor agents.

The term "chemical substitution" as used herein refers to any base, sugar and/or phosphate modification that will protect the nucleic acids from degradation by nucleases without inhibiting their catalytic activity entirely.

The term "nucleotide" is used as recognized in the art to include natural bases, and modified bases well known in the art. Such bases are generally located at the 1' position of a sugar moiety. Nucleotide generally comprise a base, sugar and a phosphate group. The nucleotides can be unmodified or modified at the sugar, phosphate and/or base moeity, (see for example, Usman and McSwiggen, *supra;* Eckstein *et al.,* International PCT Publication No. WO 92/07065; Usman *et al.,* International PCT Publication No. WO 93/15187). There are several examples of modified nucleic acid bases known in the art and has recently been summarized by Limbach *et al.,* 1994, *Nucleic Acids Res.* 22, 2183. Some of the non-limiting examples of base modifications that can be introduced into enzymatic nucleic acids without significantly effecting their catalytic activity include, inosine, purine, pyridin-4-one, pyridin-2-one, phenyl, pseudouracil, 2, 4, 6-trimethoxy benzene, 3-methyluracil, dihydrouridine, naphthyl, aminophenyl, 5-alkylcytidines (*e.g*., 5-methylcytidine), 5-alkyluridines (*e.g.*, ribothymidine), 5-halouridine (*e.g*., 5-bromouridine) or 6-azapyrimidines or 6-alkylpyrimidines (e.g. 6-methyluridine) and others (Burgin *et al.,* 1996, *Biochemistry,* 35,14090). By "modified bases" in this aspect is meant nucleotide bases other than adenine, guanine, cytosine and uracil at 1' position or their equivalents; such bases may be used within the catalytic core of the enzyme and/or in the substrate-binding regions.

There are several examples in the art describing sugar modifications that can be introduced into enzymatic nucleic acid molecules without significantly effecting catalysis and significantly enhancing their nuclease stability and efficacy. Sugar modification of enzymatic nucleic acid molecules have been extensively described in the art (see Eckstein *et al., International Publication* PCT No. WO 92/07065; Perrault *et al. Nature* 1990, 344, 565-568; Pieken *et al. Science* 1991, 253, 314-317; Usman and Cedergren, *Trends in Biochem. Sci.* 1992, 17, 334-339; Usman *et al. International Publication* PCT No. WO 93/15187; Sproat, *US Patent* No. 5,334,711 and Beigelman *et al.,* 1995 *J. Biol. Chem.* 270, 25702). Such publications describe the location of incorporation of modifications and the like. In view of such teachings, similar modifications can be used as described herein.

In a preferred embodiment the invention features a nucleic acid catalyst with non-nucleotide substitution. The non-nucleotide substitution may be in the opposite enantiomeric form as the standard non-nucleotide residue. The term "non-nucleotide" as used herein include either abasic nucleotide, polyether, polyamine, polyamide, peptide, carbohydrate, lipid or polyhydrocarbon compounds. Specific examples include those described by Seela and Kaiser, *Nucleic Acids Res.* 1990, *18*:6353 and *Nucleic Acids Res.* 1987, *15*:3113; Cload and Schepartz, *J. Am. Chem. Soc.* 1991, *113*:6324; Richardson and Schepartz, *J. Am. Chem. Soc.* 1991, *113*:5109; Ma et al., *Nucleic Acids Res.* 1993, *21*:2585 and *Biochemistry* 1993, *32*:1751; Durand et al., *Nucleic Acids Res.* 1990, *18*:6353; McCurdy et al., *Nucleosides & Nucleotides* 1991. *10*:287; Jschke et al., *Tetrahedron Lett.* 1993, *34*:301; Ono et al., *Biochemistry* 1991, *30*:9914; Arnold *et al.,* International Publication No. WO 89/02439; Usman *et al.,* International Publication No. WO 95/06731; Dudycz *et al.,* International Publication No. WO 95/11910 and Ferentz and Verdine, *J. Am. Chem. Soc.* 1991, *113*:4000. Thus, in a preferred embodiment, the invention features an enzymatic nucleic acid molecule having one or more non-nucleotide moieties, and having enzymatic activity to cleave an RNA or DNA molecule. By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine. The term "abasic" or "abasic nucleotide" as used herein encompasses sugar moieties lacking a base or having other chemical groups in place of base at the 1' position.

In preferred embodiments, the enzymatic nucleic acid includes one or more stretches of RNA, which provide the enzymatic activity of the molecule, linked to the non-nucleotide moiety. The necessary RNA components are known in the art, see, *e.g*., Usman, *supra.* By RNA is meant a molecule comprising at least one ribonucleotide residue.

As the term is used in this application, non-nucleotide-containing enzymatic nucleic acid means a nucleic acid molecule that contains at least one non-nucleotide component which replaces a portion of a ribozyme, *e.g*., but not limited to, a double-stranded stem, a single-stranded "catalytic core" sequence, a single-stranded loop or a single-stranded recognition sequence. These molecules are able to cleave (preferably, repeatedly cleave) separate RNA or DNA molecules in a nucleotide base sequence specific manner. Such molecules can also act to cleave intramolecularly if that is desired. Such enzymatic molecules can be targeted to virtually any RNA transcript.

By the phrase "nucleic acid catalyst" is meant a nucleic acid molecule capable of catalyzing a variety of reactions including the ability to repeatedly cleave other separate nucleic acid molecules (endonuclease activity) in a nucleotide base sequence-specific manner. Such a molecule with endonuclease activity may have complementarity in a substrate binding region to a specified gene target, and also has an enzymatic activity that specifically cleaves RNA or DNA in that target. That is, the nucleic acid molecule with endonuclease activity is able to intramolecularly or intermolecularly cleave RNA or DNA and thereby inactivate a target RNA or DNA molecule. This complementarity functions to allow sufficient hybridization of the enzymatic RNA molecule to the target RNA or DNA to allow the cleavage to occur. 100% complementarity is preferred, but complementarity as low as 50-75% may also be useful in this invention. The nucleic acids may be modified at the base, sugar, and/or phosphate groups. The term enzymatic nucleic acid is used interchangeably with phrases such as ribozymes, catalytic RNA, enzymatic RNA, catalytic DNA, nucleozyme, DNAzyme, RNA enzyme, endoribonuclease, endonuclease, minizyme, leadzyme, oligozyme or DNA enzyme. All of these terminologies describe nucleic acid molecules with enzymatic activity.

By "nucleic acid molecule" as used herein is meant a molecule comprising nucleotides. The nucleic acid can be composed of modified or unmodified nucleotides or non-nucleotides or various mixtures and combinations thereof.

By "complementarity" is meant a nucleic acid that can form hydrogen bond(s) with other RNA sequence by either traditional Watson-Crick or other non-traditional types (for example, Hoogsteen type) of base-paired interactions.

In preferred embodiments of this invention, the enzymatic nucleic acid molecule is formed in a hammerhead or hairpin motif, but may also be formed in the motif of a hepatitis delta virus (HDV), group I intron, RNaseP RNA (in association with an RNA guide sequence) or *Neurospora* VS RNA. Examples of such hammerhead motifs are described by Rossi *et al.,* 1992, *Aids Research and Human Retroviruses* 8, 183, of hairpin motifs by Hampel *et al.,* EP0360257, Hampel and Tritz, 1989 *Biochemistry* 28, 4929, and Hampel *et al.,* 1990 *Nucleic Acids Res.* 18, 299, and an example of the hepatitis delta virus motif is described by Perrotta and Been, 1992 *Biochemistry* 31, 16; of the RNaseP motif by Guerrier-Takada *et al.,* 1983 *Cell* 35, 849 and Forster and Altman, 1990 *Science* 249, 783, *Neurospora* VS RNA ribozyme motif is described by Collins (Saville and Collins, 1990 *Cell* 61, 685-696; Saville and Collins, 1991 *Proc. Natl. Acad. Sci. USA* 88, 8826-8830; Guo and Collins, 1995 *EMBO J.* 14, 368) and of the Group I intron by Cech *et al.,* U.S. Patent 4,987,071. These specific motifs are not limiting in the invention and those skilled in the art will recognize that all that is important in an enzymatic nucleic acid molecule with endonuclease activity of this invention is that it has a specific substrate binding site which is complementary to one or more of the target gene RNA regions, and that it have nucleotide sequences within or surrounding that substrate binding site which impart an RNA cleaving activity to the molecule.

The invention provides a method for producing a class of enzymatic cleaving agents which exhibit a high degree of specificity for the target, such as RNA of a desired target. The enzymatic nucleic acid molecule is preferably targeted to a highly conserved sequence region of a target such that specific treatment of a disease or condition can be provided with a single enzymatic nucleic acid. Such enzymatic nucleic acid molecules can be delivered exogenously to specific cells as required. In the preferred hammerhead motif the small size (less than 60 nucleotides, preferably between 30-40 nucleotides in length) of the molecule allows the cost of treatment to be reduced compared to other ribozyme motifs.

Therapeutic ribozymes must remain stable within cells until translation of the target mRNA has been inhibited long enough to reduce the levels of the undesirable protein. This period of time varies between hours to days depending upon the disease state. Clearly, ribozymes must be resistant to nucleases in order to function as effective intracellular therapeutic agents. Improvements in the chemical synthesis of RNA (Wincott *et al.,* 1995 *Nucleic Acids Res.* 23, 2677;) have expanded the ability to modify ribozymes to enhance their nuclease stability. The majority of this work has been performed using hammerhead ribozymes (reviewed in Usman and McSwiggen, 1995 *supra)* and can be readily extended to other catalytic nucleic add motifs.

By "enzymatic portion" is meant that part of the ribozyme essential for cleavage of an RNA substrate.

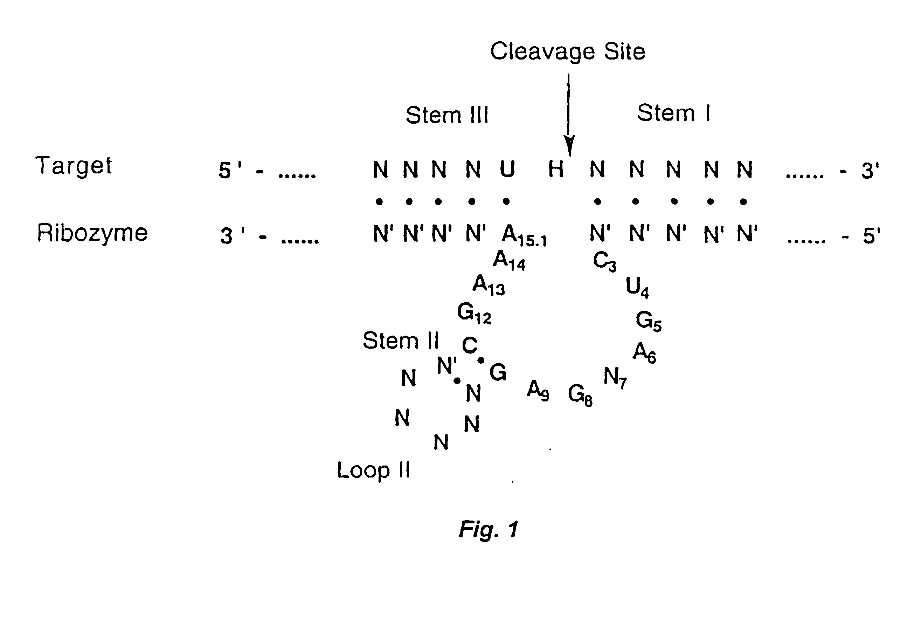

By "substrate binding arm" is meant that portion of a ribozyme which is complementary to (*i.e.,* able to base-pair with) a portion of its substrate. Generally, such complementarity is 100%, but can be less if desired. For example, as few as 10 bases out of 14 may be base-paired. Such arms are shown generally in Figures 1 as discussed below. That is, these arms contain sequences within a ribozyme which are intended to bring ribozyme and target RNA together through complementary base-pairing interactions; *e.g.,* ribozyme sequences within stems I and III of a standard hammerhead ribozyme make up the substrate-binding domain (see Figure 1).

In a preferred embodiment, the enzymatic nucleic acid molecules are added directly, or can be complexed with cationic lipids, packaged within liposomes, or otherwise delivered to smooth muscle cells. The RNA or RNA complexes can be locally administered to relevant tissues through the use of a catheter, infusion pump or stent, with or without their incorporation in biopolymers. Using the methods described herein, other enzymatic nucleic acid molecules that cleave target nucleic acid may be derived and used as described above. Specific examples of nucleic acid catalysts of the instant invention are provided below in the Tables and figures.

Sullivan, *et al., supra,* describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Sullivan *et al.,* supra and Draper *et al., supra.*

By "consists essentially of" is meant that the active ribozyme contains an enzymatic center or core equivalent to those in the examples, and binding arms able to bind target nucleic acid molecules such that cleavage at the target site occurs. Other sequences may be present which do not interfere with such cleavage.

Thus, in one aspect, the invention features ribozymes that inhibit gene expression and/or cell proliferation. These chemically or enzymatically synthesized nucleic acid molecules contain substrate binding domains that bind to accessible regions of specific target nucleic acid molecules. The nucleic acid molecules also contain domains that catalyze the cleavage of target. Upon binding, the enzymatic nucleic acid molecules cleave the target molecules, preventing for example, translation and protein accumulation. In the absence of the expression of the target gene, cell proliferation, for example, is inhibited.

The novel nucleosides or nucleotides of the instant invention and/or the polynucleotides comprising these are added directly to a cell, or can be complexed with cationic lipids, packaged within liposomes, or otherwise delivered to target cells. The nucleosides, nucleic acid or nucleic acid complexes can be locally administered to relevant tissues ex vivo, or in vivo through injection, infusion pump or stent, with or without their incorporation in biopolymers.

By "enzymatic portion" is meant that part of the ribozyme essential for cleavage of an RNA substrate.

By "substrate binding arm" is meant that portion of a ribozyme which is complementary to (*i.e.,* able to base-pair with) a portion of its substrate. Generally, such complementarity is 100%, but can be less if desired. For example, as few as 10 bases out of 14 may be base-paired. Such arms are shown generally in Figures 1-3 as discussed below. That is, these arms contain sequences within a ribozyme which are intended to bring ribozyme and target RNA together through complementary base-pairing interactions; *e.g.,* ribozyme sequences within stems I and III of a standard hammerhead ribozyme make up the substrate-binding domain (see Figure 1).

By "oligonucleotide" or "polynucleotide" as used herein, is meant a molecule comprising two or more nucleotides.

By "unmodified nucleoside" is meant one of the bases adenine, cytosine, guanine, uracil joined to the 1' carbon of β-D-ribo-furanose.

By "modified nucleoside" is meant any nucleotide base which contains a modification in the chemical structure of an unmodified nucleotide base, sugar and/or phosphate.

Oligonucleotide conjugates with different types of biologically interesting macromolecules or reporter groups are useful as experimental tools. A majority of the methods known in the art for the chemical synthesis of such conjugates are based either on post-synthetic attachment of the molecule of interest to the 3'- and/or 5'-end of oligonucleotides using appropriate spacer, or incorporation of the sugar, base and/or backbone-modified, monomeric nucleoside units into oligonucleotides during chemical synthesis. However, these methods have several disadvantages such as low yields and tedious synthesis schemes. To avoid these problems it is necessary to use unique functional groups both in the oligonucleotides and in the molecule to be attached. These functional groups should be able to react quantitatively, under mild conditions and preferably in water solution. The main idea here is to design a nucleoside monomeric unit (phosphoramidite) bearing a unique functional group, which can be further used as a tether for conjugating any molecule of interest.

Formation of oximes (interaction of aldehydes or ketones with hydroxylamines) or oxyamides (interaction of carboxylic acids with hydroxylamines) seems to be the organic reactions of choice which will meet the above requirements (Sandler, S.R.; Karo, W *Organic Functional Group Preparation* vol. III, ed. Wasserman H.H., Academic Press, Inc., 1989, pp 378-523).

In a preferred embodiment, an oligonucleotide would bear one or more hydroxylamino functionalities attached directly to the monomeric unit or through the use of an appropriate spacer Since oligonucleotides have neither aldehyde nor hydroxylamino groups, the formation of an oxime would occur selectively using oligo as a polymeric template. This approach would facilitate the attachment of practically any molecule of interest (peptides, polyamines, coenzymes, oligosaccharides, lipids, etc.) directly to the oligonucleotide using either aldehyde or carboxylic function in the molecule of interest.

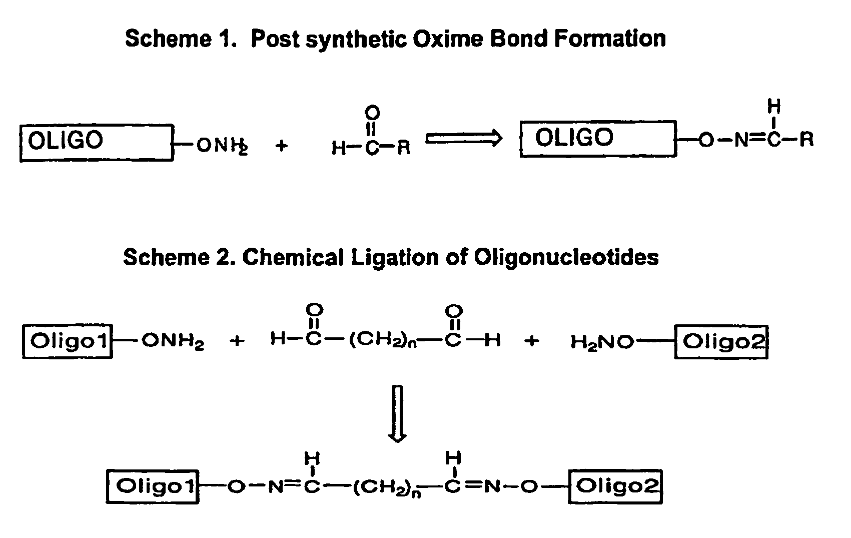

**Advantages of oxime bond formation:**
● The oximation reaction proceeds in water
● Quantitative yields
● Hydrolytic stability in a wide pH range (5 - 8)
● The amphoteric nature of oximes allows them to act either as weak acids or weak bases.
● Oximes exhibit a great tendency to complex with metal ions

In yet another preferred embodiment, the aminooxy "tether" in oligonucleotides, such as a ribozyme, is reacted with different compounds bearing carboxylic groups (e.g. aminoacids, peptides, "cap" structures ,etc.) resulting in the formation of oxyamides as shown below.

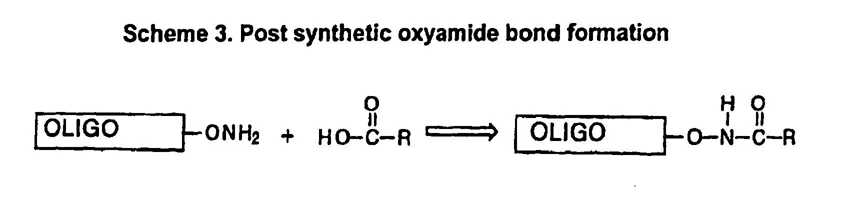

In a preferred embodiment the invention features a process for the synthesis of a 2'-*O*-amino nucleoside, such as 2'-*O*-amino adenosine, 2'-*O-*amino guanosine, 2'-*O*-amino cytidine, 2'-*O*-amino uridine and others, comprising the steps of: a) contacting a 3' and 5'-protected arabino nucleoside with sulfonylating reagent, such as tri-fluoromethane sulfonic anhydride, tri-fluoromethane sulfonic chloride and others, under conditions suitable for the formation of 3' and 5'-protected 2'-arabino sulfonyl nucleoside; b) displacement of the sulfonyl group from said 2'-arabino sulfonyl nucleoside with *N*-hydroxy-phthalimide in the presence of a strong organic base, such as 1,8-diazabicyclo(5.4.0)undec-7-ene and the like, under conditions suitable for the formation of 3' and 5'-protected 2'-*O*-*N-*phthaloyl ribonucleoside; c) deprotection of said N-phthaloyl ribonucleoside with a fluoride containing reagent, such as tetrabutylammonium fluoride, triethylamine trihydrofluoride and the like, under conditions suitable for the formation of 2'-*O*-*N*-phthaloyl ribonucleoside; and d) contacting said 2'-*O*-*N-*phthaloyl ribonucleoside with a reagent selected from a group consisting of alkylamine (such as methylamine, ethylamine, butylamine and the like), hydrazine, *N*-phenyl hydrazine and *N*-alkylhydrazine (such as *N-*methylhydrazine and the like), under conditions suitable for the formation of said 2'-O-amino nucleoside.

Other features and advantages of the invention will be apparent from the following description of the preferred embodiments thereof, and from the claims.

### Description of the Preferred Embodiments

The drawings will first briefly be described.

### Drawing:

Figure 1 is a diagrammatic representation of the hammerhead ribozyme domain known in the art. Stem II can be ≥ 2 base-pair long. Each N is independently any base or non-nucleotide as used herein.
Figure 2a is a diagrammatic representation of the hammerhead ribozyme domain known in the art; Figure 2b is a diagrammatic representation of the hammerhead ribozyme as divided by Uhlenbeck (1987, *Nature,* 327, 596-600) into a substrate and enzyme portion; Figure 2c is a similar diagram showing the hammerhead divided by Haseloff and Gerlach (1988, *Nature,* 334, 585-591) into two portions; and Figure 2d is a similar diagram showing the hammerhead divided by Jeffries and Symons (1989, *Nucl. Acids. Res.,* 17, 1371-1371) into two portions.

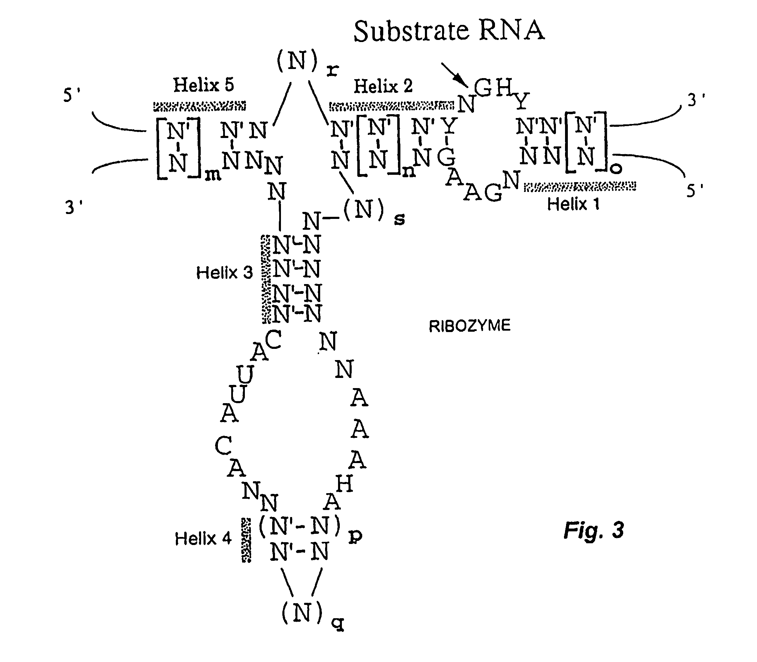

Figure 3 is a diagrammatic representation of the general structure of a hairpin ribozyme. Helix 2 (H2) is provided with at least 4 base pairs (*i.e.,* n is 1, 2, 3 or 4) and helix 5 can be optionally provided of length 2 or more bases (preferably 3 - 20 bases, *i.e.,* m is from 1 - 20 or more). Helix 2 and helix 5 may be covalently linked by one or more bases (*i.e.,* r is ≥ 1 base). Helix 1, 4 or 5 may also be extended by 2 or more base pairs (*e.g.,* 4 - 20 base pairs) to stabilize the ribozyme structure, and preferably is a protein binding site. In each instance, each N and N' independently is any normal or modified base and each dash represents a potential base-pairing interaction. These nucleotides may be modified at the sugar, base or phosphate. Complete base-pairing is not required in the helices, but is preferred. Helix 1 and 4 can be of any size (*i.e.,* o and p is each independently from 0 to any number, *e.g*., 20) as long as some base-pairing is maintained. Essential bases are shown as specific bases in the structure, but those in the art will recognize that one or more may be modified chemically (abasic, base, sugar and/or phosphate modifications) or replaced with another base without significant effect. Helix 4 can be formed from two separate molecules, *i.e.,* without a connecting loop. The connecting loop when present may be a ribonucleotide with or without modifications to its base, sugar or phosphate. "q" is ≥ 2 bases. The connecting loop can also be replaced with a non-nucleotide linker molecule. H, refers to bases A, U or C. Y refers to pyrimidine bases. "―" refers to a chemical bond.
Figure 4 is a representation of the general structure of the hepatitis delta virus ribozyme domain known in the art. Each N is independently any base or non-nucleotide as used herein.
Figure 5 is a representation of the general structure of the self-cleaving VS RNA ribozyme domain.
Figure 6 is a diagrammatic representation of a few of the novel nucleoside analogs of the instant invention. **1**,3-(β-D-Ribofuranosyl)-pyridin-2-one; **2**, 5-(β-D-Ribofuranosyl)-pyridin-2-one; **3**, 5-(β-D-Ribofuranosyl)-2-bromopyridine; **4**, 5-(β-D-Ribofuranosyl)-2-aminopyridine; **5**, 3-(β-D-Ribofuranosyl)-2-fluoropyridine; **6**,3-(α-D-Ribofuranosyl)-2-fluoropyridine.
Figure 7 is a scheme for the synthesis of 5-(β-D-Ribofuranosyl)-pyridin-2-one (**2** in Figure 6) and 5-(β-D-Ribofuranosyl)-2-bromopyridine (3 in Figure 6 and **11** in Figure 7) monomers.
Figure 8 describes two schemes for the synthesis of 3-(β-D-Ribofuranosyl)-pyridin-2-one (**1** in Figure 6 and **13** in Figure 8B) and 3-(β-D-Ribofuranosyl)-2-fluoropyridine (**5** in Figure 6 and **12** in Figure 8B) monomers.
Figure 9 is a scheme for the synthesis of 1-(5-*O*-Dimethoxytrityl-2-*O-*tert-butyldimethylsilyl-3-O-2-cyanoethyl-N,N- diisopropylaminophosphoramidite-β-D-ribofuranosyl)-1,4-dihydro-pyrimidine-4-one.
Figure 10 is a scheme for the synthesis of 1-(5-*O*-Dimethoxytrityl-2-*O-*tert-butyldimethylsilyl-3-O-2-cyanoethyl-N,N- diisopropylaminophosphoramidite-β-D-Ribofuranosyl)-1,4-dihydropyrimidine-2-one.
Figure 11 is a scheme for the synthesis of 5'-*O*-Dimethoxytrityl-2'-*O* -tert-Butyldimethylsilyl-O⁴-Diphenylcarbamoyl-3-Deaza Uridine 3'-(2-Cyanoethyl N,N-diisopropylphosphoramidite).
Figure 12 is a scheme for the synthesis of 5'-*O*-dimethoxytrityl-2'-*O* -tert-butyldimethylsilyl-O⁴-diphenylcarbamoyl-3-deaza cytidine 3'-(2-Cyanoethyl N,N-diisopropylphosphoramidite).
Figure 13 is a scheme for the synthesis of 2-*O*-*t*-Butyldimethylsilyl-5-*O*-Dimethoxytrityl-3-*O*-(2-Cyanoethyl-*N,N*-diisopropylphosphoramidite)-_β-D-ribofuranosylindole.
Figure 14 is a scheme for the synthesis of 2-*O*-*t*-Butyldimethylsilyl-5-*O*-Dimethoxytrityl-3-*O*-(2-Cyanoethyl-*N,N*-diisopropylphosphoramidite)-_β-D-ribofuranosylbenzimidazole.
Figure 15 is a scheme for the synthesis of 3-(β-D-Ribofuranosyl)-Pyridine-2-(4-Nitrophenylethyl)-Oneand5-(β-D-Ribofuranosyl)-Pyridine-2-(4-Nitrophenylethyl)-One Phosphoramidite.
Figure 16 is a scheme for the synthesis of 2'-*O*-amino pyrimidine nucleoside and phosphoramidite.
Figure 17 is a scheme for the synthesis of 2'-*O*-amino adenosine nucleoside and phosphoramidite.
Figure 18 is a scheme for the synthesis of 2'-*O*-amino guanosine nucleoside and phosphoramidite.
Figure 19 is a diagrammatic representation of hammerhead ribozymes substituted with 2'-*O*-amino groups at various positions (A). (B) shows the rates of RNA cleavage reaction catalyzed by the ribozymes of 9 A.

### Synthesis of Polynucleotides

Nucleic acid molecules, for example having an endonuclease enzymatic activity are able to repeatedly cleave other separate RNA molecules in a nucleotide base sequence-specific manner. Such enzymatic RNA molecules can be targeted to virtually any RNA transcript, and efficient cleavage achieved in vitro (Zaug *et al.,* 324, *Nature* 4291986 ; Kim et al., 84 Proc. Natl. Acad. Sci. USA 8788,1987; Haseloff and Gerlach, 334 Nature 585, 1988; Cech, 260 JAMA 3030, 1988; and Jefferies et al., 17 Nucleic Acids Research 1371, 1989). Because of their sequence-specificity, trans-cleaving ribozymes show promise as therapeutic agents for human disease (Usman & McSwiggen, 1995 *Ann. Rep. Med. Chem.* **30,** 285-294; Christoffersen and Marr, 1995 *J. Med. Chem.* **38,** 2023-2037). Ribozymes can be designed to cleave specific RNA targets within the background of cellular RNA. Such a cleavage event renders the mRNA non-functional and abrogates protein expression from that RNA. In this manner, synthesis of a protein associated with a disease state can be selectively inhibited.

Seven basic varieties of naturally-occurring enzymatic RNAs are known presently. Each can catalyze the hydrolysis of RNA phosphodiester bonds in *trans* (and thus can cleave other RNA molecules) under physiological conditions. Table I summarizes some of the characteristics of these ribozymes. In general, enzymatic nucleic acids act by first binding to a target RNA. Such binding occurs through the target binding portion of a enzymatic nucleic acid which is held in close proximity to an enzymatic portion of the molecule that acts to deave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over other technologies, since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base-pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base-pairing. Thus, it is thought that the specificity of action of a ribozyme is greater than that of antisense oligonucleotide binding the same RNA site.

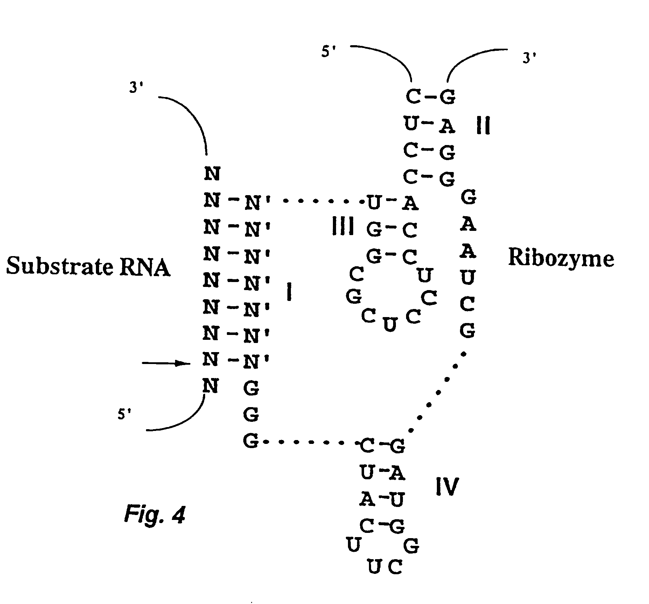

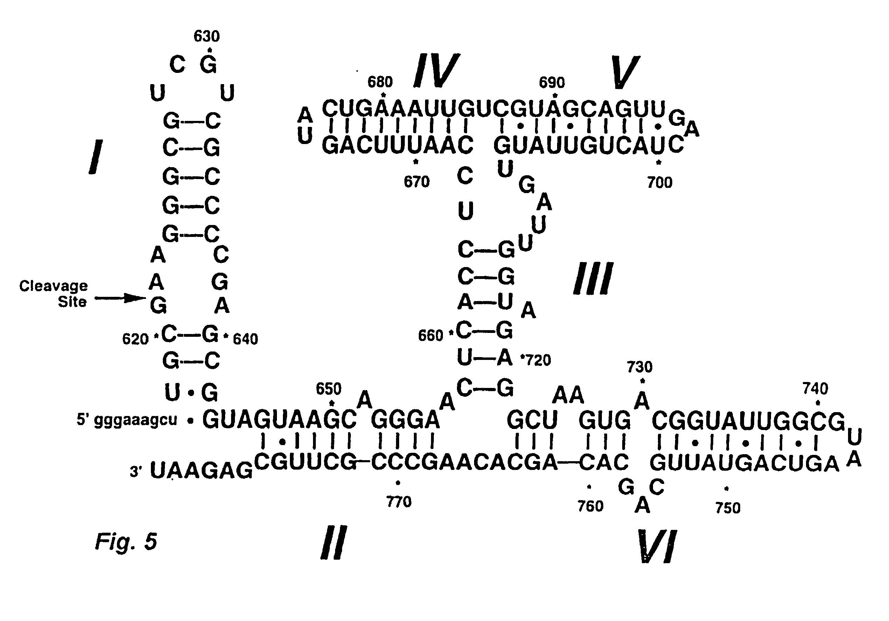

In one of the preferred embodiments of the inventions herein, the enzymatic nucleic acid molecule is formed in a hammerhead or hairpin motif, but may also be formed in the motif of a hepatitis δ virus, group I intron, group ii intron or RNaseP RNA (in association with an RNA guide sequence) or *Neurospora* VS RNA (figure 4, 5). Examples of such hammerhead motifs are described by Dreyfus, *supra,* Rossi *et al.,* 1992, *AIDS Research and Human Retroviruses 8,* 183; of hairpin motifs by Hampel *et al.,* EP0360257, Hampel and Tritz, 1989 *Biochemistry* 28,4929, Feldstein *et al.,* 1989, *Gene* 82, 53, Haseloff and Gerlach, 1989, *Gene,* 82, 43, and Hampel *et al.,* 1990 *Nucleic Acids Res.* 18, 299; of the hepatitis δ virus motif is described by Perrotta and Been, 1992 *Biochemistry* 31, 16; of the RNaseP motif by Guerrier-Takada *et al.,* 1983 *Cell* 35, 849; Forster and Altman, 1990, *Science* 249, 783; Li and Altman, 1996, *Nucleic Acids Res.* 24, 835; *Neurospora* VS RNA ribozyme motif is described by Collins (Saville and Collins, 1990 *Cell* 61, 685-696; Saville and Collins, 1991 *Proc. Natl. Acad. Sci. USA* 88, 8826-8830; Collins and Olive, 1993 *Biochemistry* 32, 2795-2799; Guo and Collins, 1995, *EMBO. J.* 14, 363); Group II introns are described by Griffin *et al.,* 1995, *Chem. Biol.* 2, 761; Michels and Pyle, 1995, *Biochemistry* 34, 2965; Pyle *et al.,* International PCT Publication No. *WO 96*/*22689*; and of the Group I intron by Cech *et al.,* U.S. Patent 4,987,071. These specific motifs are not limiting in the invention and those skilled in the art will recognize that all that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule.

Synthesis of polynucleotides greater than 100 nucleotides in length is difficult using automated methods, and the therapeutic cost of such molecules is prohibitive. In this invention, small enzymatic nucleic acid motifs (*e.g*., of the hammerhead or the hairpin structure) are used for exogenous delivery. The simple structure of these molecules increases the ability of the enzymatic nucleic acid to invade targeted regions of the mRNA structure.

RNA molecules, such as the ribozymes are chemically synthesized. The method of synthesis used follows the procedure for normal RNA synthesis as described in Usman *et al.,* 1987 *J. Am. Chem. Soc.,* 109, 7845; Scaringe *et al.,* 1990 *Nucleic Acids Res.,* 18, 5433; and Wincott *et al.,* 1995 *Nucleic Acids Res.* 23, 2677-2684 and makes use of common nucleic acid protecting and coupling groups, such as dimethoxytrityl at the 5'-end, and phosphoramidites at the 3'-end. Small scale synthesis were conducted on a 394 Applied Biosystems, Inc. synthesizer using a modified 2.5 µmol scale protocol with a 5 min coupling step for alkylsilyl protected nucleotides and 2.5 min coupling step for 2'-*O*-methylated nucleotides. Table 2 outlines the amounts, and the contact times, of the reagents used in the synthesis cycle. A 6.5-fold excess (163 µL of 0.1 M = 16.3 µmol) of phosphoramidite and a 24-fold excess of *S*-ethyl tetrazole (238 µL of 0.25 M = 59.5 µmol) relative to polymer-bound 5'-hydroxyl was used in each coupling cycle. Average coupling yields on the 394 Applied Biosystems, Inc. synthesizer, determined by colorimetric quantitation of the trityl fractions, were 97.5-99%. Other oligonucleotide synthesis reagents for the 394 Applied Biosystems, Inc. synthesizer: detritylation solution was 2% TCA in methylene chloride (ABI); capping was performed with 16% *N*-methyl imidazole in THF (ABI) and 10% acetic anhydride/10% 2,6-lutidine in THF (ABI); oxidation solution was 16.9 mM I₂, 49 mM pyridine, 9% water in THF (Millipore). B & J Synthesis Grade acetonitrile was used directly from the reagent bottle. S-Ethyl tetrazole solution (0.25 M in acetonitrile) was made up from the solid obtained from American International Chemical, Inc.

Deprotection of the RNA was performed as follows. The polymer-bound oligoribonucleotide, trityl-off, was transferred from the synthesis column to a 4mL glass screw top vial and suspended in a solution of methylamine (MA) at 65 °C for 10 min. After cooling to -20 °C, the supernatant was removed from the polymer support. The support was washed three times with 1.0 mL of EtOH:MeCN:H₂O/3:1:1, vortexed and the supernatant was then added to the first supernatant. The combined supernatants, containing the oligoribonucleotide, were dried to a white powder.

The base-deprotected oligoribonucleotide was resuspended in anhydrous TEA●HF/NMP solution (250 µL of a solution of 1.5mL *N*-methylpyrrolidinone, 750 µL TEA and 1.0 mL TEA●3HF to provide a 1.4M HF concentration) and heated to 65°C for 1.5 h. The resulting, fully deprotected, oligomer was quenched with 50 mM TEAB (9 mL) prior to anion exchange desalting.

For anion exchange desalting of the deprotected oligomer, the TEAB solution was loaded onto a Qiagen 500® anion exchange cartridge (Qiagen Inc.) that was prewashed with 50 mM TEAB (10 mL). After washing the loaded cartridge with 50 mM TEAB (10 mL), the RNA was eluted with 2 M TEAB (10 mL) and dried down to a white powder.

Inactive hammerhead ribozymes were synthesized by substituting a U for G₅ and a U for A₁₄ (numbering from (Hertel, K. J., et *al.,* 1992, *Nucleic Acids Res.,* 20, 3252)).

The average stepwise coupling yields were >98% (Wincott *et al.,* 1995 *Nucleic Acids Res.* 23, 2677-2684).

Hairpin ribozymes are synthesized either as one part or in two parts and annealed to reconstruct the active ribozyme (Chowrira and Burke, 1992 *Nucleic Acids Res.,* 20, 2835-2840).

RNAs are purified by gel electrophoresis using general methods or are purified by high pressure liquid chromatography (HPLC; See Stinchcomb *et al.,* International PCT Publication No. WO 95/23225), and are resuspended in water.

Various modifications to ribozyme structure can be made to enhance the utility of ribozymes. Such modifications will enhance shelf-life, half-life in vitro, stability, and ease of introduction of such ribozymes to the target site, e.g., to enhance penetration of cellular membranes, and confer the ability to recognize and bind to targeted cells.

### Optimizing Ribozyme Activity

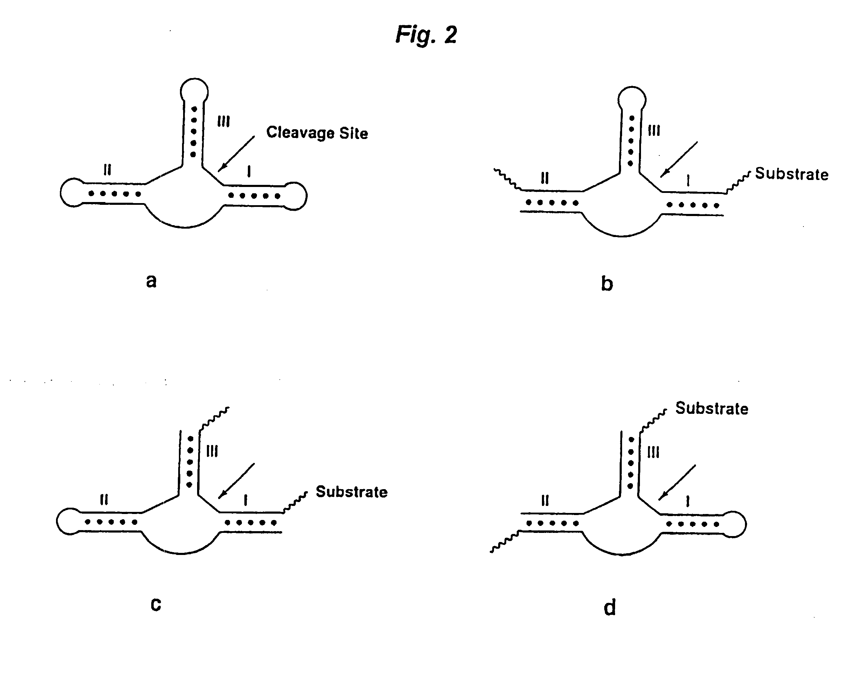

Ribozyme activity can be optimized as described by Stinchcomb *et al.,* supra. The details will not be repeated here, but include altering the length of the ribozyme binding arms (stems I and III, see Figure 2c), or chemically synthesizing ribozymes with modifications that prevent their degradation by serum ribonucleases (see *e.g.,* Eckstein *et al.,* International Publication No. WO 92/07065; Perrault *et al.,* 1990 *Nature* 344**,** 565; Pieken *et al.,* 1991 *Science* 253, 314; Usman and Cedergren, 1992 *Trends in Biochem. Sci.* 17**,** 334; Usman *et al.,* International Publication No. WO 93/15187: Rossi *et al.,* International Publication No. WO 91/03162; as well as Sproat, US Patent No. 5,334,711 which describe various chemical modifications that can be made to the sugar moieties of enzymatic RNA molecules). Modifications which enhance their efficacy in cells, and removal of stem II bases to shorten RNA synthesis times and reduce chemical requirements are desired.

### Administration of Polynucleotides

Sullivan *et al.,* PCT WO 94/02595, describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Sullivan *et al.,* supra and Draper *et al.,* PCT WO93/23569.

### Example

The following are non-limiting examples showing the synthesis and activity of the certain nucleosides/nucleotides of the instant invention and polynucleotides comprising one or more of these. Those in the art will recognize that certain reaction conditions such as temperatures, pH, ionic conditions, reaction times and solvent conditions described in the following examples are not meant to be limiting and can be readily modified without significantly effecting the synthesis.

### Example 1: Synthesis of Ribozymes Containing Base-Modified Nucleotides

The method of synthesis used follows the procedure for normal RNA synthesis as described in Usman *et al., J. Am. Chem. Soc.* 1987, *109*, 7845-7854; Scaringe *et al., Nucleic Acids Res.* 1990, *18,* 5433-5441 and Wincott *et al.,* 1995 *Nucleic Acids Res.* 23, 2677-2684 and makes use of common nucleic acid protecting and coupling groups, such as dimethoxytrityl at the 5'-end, and phosphoramidites at the 3'-end (compounds 4, 9, 13, 17, 22, 23). The average stepwise coupling yields were >98%. These base-modified nucleotides may be incorporated not only into hammerhead ribozymes, but also into hairpin, VS ribozymes, hepatitis delta virus, or Group I or Group II introns. They are, therefore, of general use as replacement motifs in any nucleic acid structure.

### Example 2: Synthesis of 5-(β-D-Ribofuranosyl) -Pyridin-2-one (2)

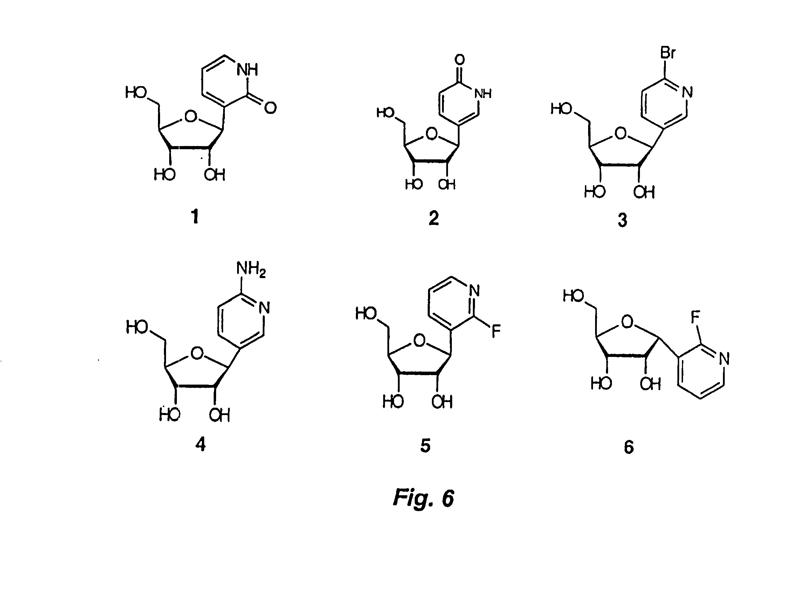

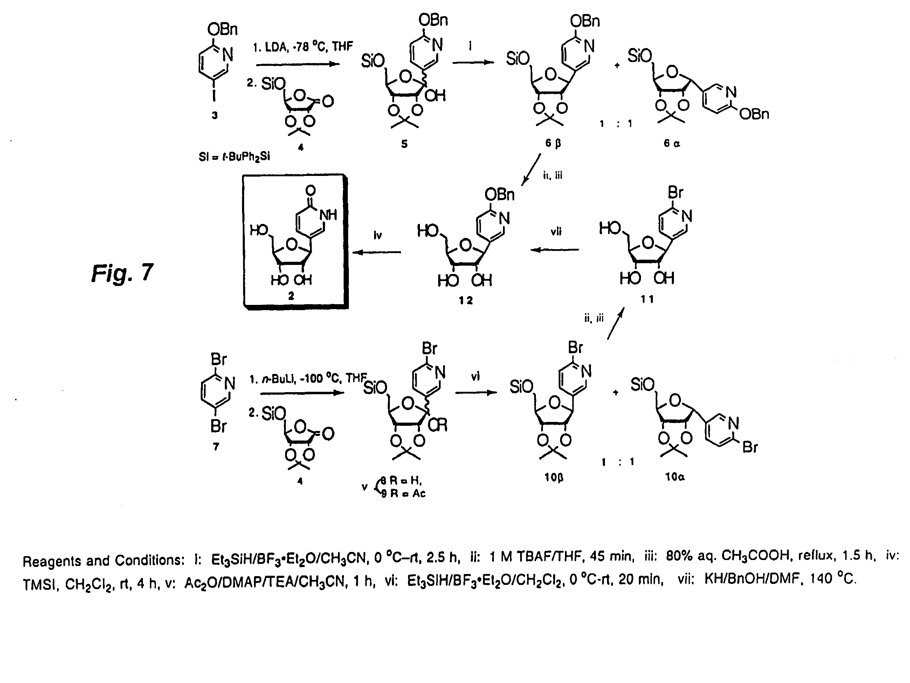

Referring to Figure 7, Pyridin-2-one C-nucleoside **2** (Figure 6) was prepared in 5 steps from 2-(benrytoxy)-5-iodopyridine (**3**) and D-ribono-1,4-lactone **4** and alternatively, in 7 steps from 2,5-dibromopyridine (7) and D-ribono-1,4-lactone **4**. The intermediate 1'-O-Ac derivative **9** was crucial for the successful deoxygenation of hemiacetal **8**.

The synthesis of a pyrimidine nucleoside analog **2** which lacks O² carbonyl is described herein. These analogs of pyrimidine nucleosides with novel H-bonding patterns could serve as valuable tools for identification of essential intramolecular hydrogen bonding interactions. 2'-Deoxy analog of **1** (Solomon, M.S.; Hopkins, P.B. *Tetrahedron Lett.* **1991,** *32*, 3297-3300; Solomon, M.S.; Hopkins, P.B. *J*. *Org. Chem.* **1993,** *58,* 2232-2243) and **1** is described as a mixture of α and β anomers (Belmas *et al.,* 1989 *Nucleosides & Nucleotides* 8, 307). The ribo derivative **2** is not known and is described here.

In one attempt to synthesize **2** (Figure 7) Applicant converted 2-(benzyloxy)-5-iodopyridine (**3**) (synthesized from 2-bromo-5-iodopyridine (Hama *et al., Bull. Chem. Soc. Jpn.* **1988,** *61,* 1683-1686) using reducing reagents such as NaH/BnOH/DMF) to 5-lithio derivative by metallation using reagents such as, lithium diazapropylamine (LDA) under suitable conditions such as using a temperature of about -78 °C. Condensation of a protected D-ribonolactone, such as 5-*O*-*t*-butytdiphenylsilyl-2,3-*O*-isopropylidene-D-ribonolactone (**4**) with this intermediate (5-lithio derivative) yielded the mixture of α and β lactols **5** in 1:8 ratio (47% yield). It should be noted that for hemiacetals **5, 8** and **9,** the prefix a refers to the position of the glycosidic OH group relative to the configuration at the reference C-atom (C4' in **2;** *i.e.* the pyridyl moiety is in the β-position). The assignments of the anomeric configurations were based on Δδ values for the isopropylidene Me groups in ¹H NMR spectra. It is known from the previous work (Gudmundsson *et al., Tetrahedron Lett.* **1996,** *37*, 2365-2368; Dondoni, A.; Scherrmann, M.-C. *J. Org. Chem.* **1994,** *59*, 6404-6412) that reductive 1'-deacetoxylation is a much more efficient approach to 1'-deoxygenation than direct reduction of 1'-OH. Unfortunately, acetylation of **5** with Ac₂O/DMAP/TEA in acetonitrile failed; the major product did not contain acetyl group by ¹H NMR.

Reduction of **5** with reducing agents, such as triethylsilane (Et₃SiH)/BF₃●Et₂O in acetonitrile at temperatures such as room temperature yielded an 1:1 α/β mixture of C-nucleosides **6** in 21% yield. 6β and 6α were easily separated by flash column chromatography using 5-10% gradient of methanol in dichloromethane for elution.

6β was deblocked using reagents such as TBAF to remove the 5'-silyl ether protection followed by the cleavage of isopropylidene group with acid to yield **12** in a good yield (¹H NMR (CD₃)₂CO + D₂O) data for **12**: δ 8.19 (d, J_{6,4}=2.4, 1H, H6), 7.79 (dd,J_{4,3}=8.4, J_{4,6}=2.4, 1H, H4), 6.80 (d, J_{3,4}=8.4, 1 H, H3), 5.37 (s, 2H, CH₂Ph), 4.64 (d, J_{1',2'}=7.2, 1H, H1'), 4.12 (dd,J_{3',2'}=5.4, J_{3',4'}=3.4, 1H, H3'), 3.95 (m, 1H, H4'), 3.88 (dd, J_{2',1'}=7.2, J_{2',3'}=5.4, 1H, H2'), 3.74 (dd, J_{5',4'}=3.6, J_{5',5"}=12.0, 1H, H5'), 3.69 (dd, J_{5",4'}=4.2, J _{5",5'}=12.0, 1H, H5").

Compound **12** was obtained by removing the Bzl group using a variety of different approaches, such as cleavage of benzyl ether group of **12** using trimethylsilyl iodide (TMSI) in dichloromethane yielding the free C-nucleoside **2** in a good yield.

Parham and Piccirilli *(J. Org. Chem.* **1977**, *42*, 257-260) described an unexpected and highly selective halogen-lithium exchange between 2,5-dibromopyridine (**7**) and *n*-butyllithium at very low temperature (-100 °C), where only 5-bromo substituent was exchanged. Applicant synthesized **2** by an alternative approach starting from **7** and D-ribono-1,4-lactone **4** (Scheme 1). Metallation of **7** at about -100°C in THF and condensation of the resulting lithiated pyridine with protected D-ribono-1,4-lactone **4** at -100 °C afforded the expected hemiacetal **8** (44% yield, α/β 1:12) along with two by-products in 6% and 17% yield, respectively.

Procedure for the preparation of **8**: 2,5-dibromopyridine **7** (1.75 g, 7.4 mmol) was dissolved in dry THF (46 ml) under argon and the solution was cooled to -100 °C. 1.6 M n-BuLi in hexanes (5.09 ml, 8.14 mmol) was added dropwise over 5 min and the solution stirred at -100 °C for 30 min. The solution of D-ribono-1,4-lactone **4** (3 g, 7 mmol) in dry THF (10 ml) was added dropwise over 5 min and the mixture warmed up to rt over 40 min. It was stirred additional 20 min at rt , than quenched with saturated aq. NH₄Cl. The mixture was extracted with ether, organic layer washed with brine, dried and evaporated to a syrup. Flash column chromatography using 3-15% gradient of ethyl acetate in hexanes eluted first **8** (1.8 g, 44%), followed by the slower product (0.23 g, 6%). At the end the slowest material eluted (0.7 g, 17%).

All three products exhibited sugar ¹H NMR signals consistent with the hemiacetal structure. This result demonstrates the selectivity in lithiation of **7,** but the formation of other products suggests that competing halogen-lithium exchange occurred, too. Deoxygenation of **8** using the same procedure as for reduction of **5** yielded, similarly as in the case of **5,** an **1:1** mixture of α/β nucleosides in a low yield. On the other hand, acetylation of **8** proceeded in a quantitative yield to give **9** (only β-anomer was obtained). Reduction of **9** using Et₃SiH/BF₃●Et₂O/CH₂Cl₂ at 0 °C-rt proceeded in 82% yield but again without selectivity (**10**α/**10**β 1:1). The anomeric assignments were based on, besides Δδ of methyl groups, on the well known upfield shift of the 1'-H signal for the β-anomer compared to the α-anomer (Tam *et al., J. Org. Chem.* **1979,** *44,* 4854-4862; Sokolova *et al., Carbohydr. Res.* **1981,** *93,* 19-34). 10β was deprotected to **11** in two steps (1 M TBAF, followed by refluxing 80% acetic acid). Derivative **11** was highly resistant to displacement of 2-bromo substituent with methoxide and benzylate; only KH/BnOH/DMF at 140 °C afforded **12** in a good yield, identical in every aspect with the compound synthesized from 2-(benzyloxy)-5-iodopyridine **3**. This comparison proved unequivocally that 5-pyridyl regioisomer **8** was the main product obtained from 2,5-dibromopyridine **7**.

*Synthesis of 5-(α-D-Ribofuranosyl)-pyridin-2-one:* Treatment of **6**α with TBAF to remove the 5'-silyl ether protection followed by the cleavage of isopropylidene group with acid followed by cleavage of benzyl ether group using trimethylsilyl iodide (TMSI) in dichloromethane will yield 5-(α-D-Ribofuranosyl)-pyridin-2-one in a good yield.

*Synthesis of 5-(α-D-Ribofuranosyl)-2-bromopyridine*: Treatment of 10α with TBAF to remove the 5'-silyl ether protection followed by the cleavage of isopropylidene group with acid will yield 5-(α-D-Ribofuranosyl)-2-bromopyridine in good yield.

*Synthesis of 5-(β-D-Ribofuranosyl)-2-aminopyridine:* 2-Bromo derivative **11** (580 mg, 2 mmol) was placed in a steel autoclave and the autoclave purged with a continuous stream of Ar. Cul (720 mg, 2 eq.) was added and the autoclave cooled in a Dewar vessel (CO₂-isopropanol). Purging over the apparatus with Arwas continued while NH₃ was condensed in the autoclave (ca. 15 ml). The autoclave was then quickly closed, the mixture allowed to warm to rt, placed in a heated oil bath at 115 °C and stirred for 24 h. The autoclave was then cooled as before, opened, warmed up to rt and NH₃ evaporated. Then, the dark mixture was dissolved in MeOH, filtered and evaporated to a syrup. Column chromatography (silica gel, MeOH/CHCl₃) yielded *5-(β-D-Ribofuranosyl)-2-aminopyridine* (**4** in Figure 6) (235 mg, 52%).

*Synthesis of 5-(α-D-Ribofuranosyl)-2-aminopyridine:* 5-(α-D-Ribofuranosyl)-2-bromopyridine (580 mg, 2 mmol) was placed in a steel autoclave and the autoclave purged with a continuous stream of Ar. Cul (720 mg, 2 eq.) was added and the autoclave cooled in a Dewar vessel (CO₂-isopropanol). Purging over the apparatus with Ar was continued while NH₃ was condensed in the autoclave (*ca*. 15 ml). The autoclave was then quickly closed, the mixture allowed to warm to rt, placed in a heated oil bath at 115 °C and stirred for 24 h. The autoclave was then cooled as before, opened, warmed up to rt and NH₃ evaporated. Then, the dark mixture was dissolved in MeOH, filtered and evaporated to a syrup. Column chromatography (silica gel, MeOH/CHCl₃) yielded *5-(α-D-Ribofuranosyl)-2-aminopyridine.*

Although the two approaches reported herein produced α/β anomers in 1:1 ratio, they represent useful methods for the preparation of larger quantities of C-nucleoside **2** for the synthesis of oligonucleotide building blocks. Both **2** and **11** might display, like other C-nucleosides, pharmaceutically useful biological activities.

### Example 3: Synthesis of 3-(β-D-Ribofuranosyl)-2-Fluoropyridine (1) and 3-(β-D-Ribofuranosyl)-Pyridin-2-one (5)

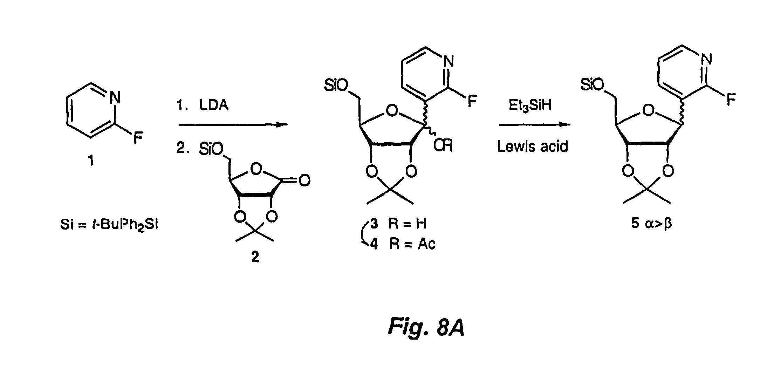

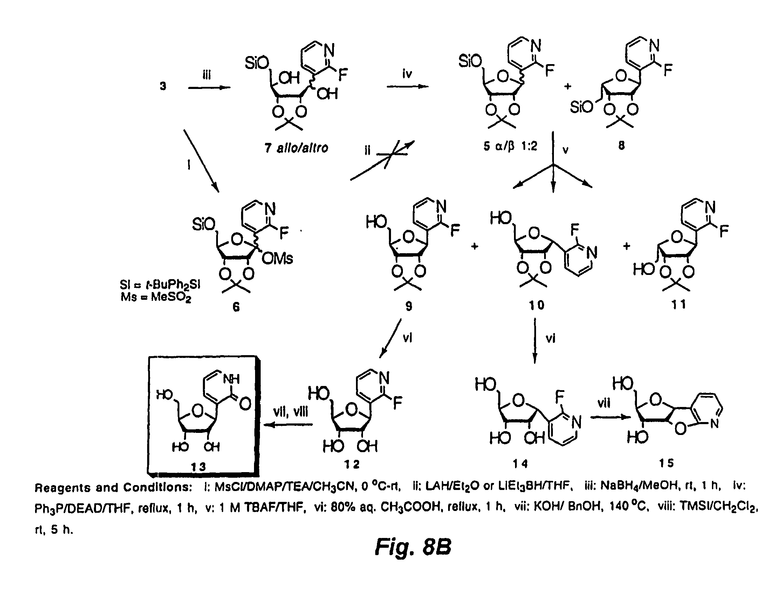

Referring to Figure 8, Pyridin-2-one C-nucleoside **13** (**1** in Figure **6**) was synthesized by reducing **3** at room temperature followed by a cyclization reaction to yield compounds **5** and **8.** Deblocking followed by hydrogenolysis and debenzylation yields compound **13.**

Pyridin-2-one C-nucleoside **13** (**1** in Figure 6) was prepared in **7** steps from 2-fluoro-3-lithiopyridine (**1**) and D-ribono-1,4-lactone **2.** The successful approach to β-ribofuranosides **12** (**5** in Figure **6**) and **13** consisted in the reductive opening of furanose ring of hemiacetal **3** followed by intramolecular Mitsunobu cyclization.

The synthesis of a pyrimidine nucleoside analogs **12** and **13** which lack O⁴ carbonyl is described herein and can be used for identification of essential intramolecular hydrogen bonding interactions.

The synthesis of **13** as an anomeric mixture is described from 2,4:3,5-di-O-benzylidene-aldehydo-D-ribose and 3-lithio-2-fluoropyridine (Belmans *et al., Nucleosides & Nucleotides* **1989,** *8*, 307-315). This procedure, however, was inefficient and not amenable to large-scale preparations due to involvement of a ribose dithioacetal during preparation of the key starting material. Besides, 2-fluropyridine derivative **12** could not be isolated by this route because of the solvolytic displacement of fluorine atom during the acid catalyzed cyclization of open chain precursor.

Referring to Figure 8A, Applicant used a protected D-ribonolactone such as 5-*O*-*t*-butyldiphenylsilyl-2,3-*O*-isopropylidene-D-ribonolactone (**2**) in the condensation with 3-lithio-2-fluoropyridine (-78 °C, than rt, 18 h, THF) and obtained 1:5 α/β mixture of lactols **3** in 63 % yield (Scheme 1). It should be noted that for hemiacetals **3**, the prefix α refers to the position of the glycosidic OH group relative to the configuration at the reference C-atom (C4' in **2**; *i.e.* the pyridyl moiety is in the β-position). The assignments of the anomeric configurations were based on Δδ values for the isopropylidene Me groups in ¹H NMR spectra. All attempts to dehydroxylate **3** using triethylsilane (Et₃SiH) in the presence of BF₃●Et₂O or TMSOTf in a variety of solvents resulted in poor yields of nucleosides **5**. Besides, the desired β-nucleoside was always the minor anomer in the mixture. The anomeric assignments were based on, besides Δδ of isopropylidene methyl groups, on the well known upfield shift of the 1'-H signal for the β-anomer compared to the α-anomer.

It was demonstrated that the more efficient and/or selective reduction of 1'-hydroxyl group can be achieved through intermediate 1'-OAc derivative (*i.e.* **4;** Gudmundsson *et al., Tetrahedron Lett.* **1996,** *37*, 2365; Dondoni *et al, J. Org. Chem.* **1994,** *59,* 6404-6412). Applicant found that **3** could be acetylated in a quantitative yield using Ac₂O/TEA/DMAP in acetonitrile to give **4** (only one isomer was generated, no attempt was made to determine its configuration). Reductive deacetoxylation of **4** with Et₃SiH/TMSOTf in dichloromethane at 0 °C-rt proceeded efficiently and afforded a good yield of **5** (α/β 10:1); when Et₃SiH was a solvent the α/β ratio was 5:1. These results are surprising because under similar reaction conditions the exclusive β-selectivity in the reduction of pyrazine (Liu *et al., Tetrahedron Lett*. **1996,** *37,* 5325-5328) and imidazo[1,2-a]pyridine lactols (Gudmundsson *et al., Tetrahedron Lett.* **1996,** *37*, 2365-2368) was reported.

In order to obtain practical quantities of β C-nucleoside Applicant investigated further several different pathways: Mesylation of **3** afforded 1'-*O*-mesyl derivative **6** in a moderate yield (Scheme 2). Treatment of 6 with LAH did not reduce the sulfonate as reported for 2-pyridine C-nucleosides. The more reactive LiEt₃BH also failed. It is worth noting that preparation of a more reactive 1'-*O*-Tf derivative failed due to the opening of the furanose ring under triflylation conditions.

Pankiewicz *et al.,* (*J. Org. Chem.* **1988**, *53*, 3473-3479) reported hydrogenolytic opening of the hemiacetal ring of 6-pyridine lactol derivatives to generate mixtures of *allo* and *altro* isomers. Reaction of **3** with NaBH₄ proceeded in a quantitative yield to give ca 1:1 ratio of *allo*/*altro* **7**. These epimers were not separated but treated under standard Mitsunobu conditions [diethyl azodicarboxylate (DEAD)/Ph₃P/THF, reflux] to give **5** (α/β ratio 1:2) and 3-(2,3-*O*-isopropylidene-5-*O*-*t*-butyldiphenylsilyl-α-L-*lyxo-*furanosyl)-2-fluoropyridine (**8**), the latter arising from the competitive formation of 4'-oxyphosphonium intermediate. Yokoyama *et al.,* (*Chem. Lett.* **1994,** 265-268) speculated that such an intermediate is formed because of the hydrogen bonding between 1'-OH and the hydrogen acceptor on the 2-pyridyl base. It is worth noting that if Mitsunobu cyclization of **7** was conducted at rt more *lyxo* derivative **8** was obtained than at reflux temperature. This indicates the existence of the hydrogen bond between 1'-OH and fluorine in its close proximity which could be disrupted by increasing the reaction temperature. Recently, an efficient synthesis of imidazole C-nucleosides was reported using similar Mitsunobu cyclization as the key step (Hurusawa *et al., J. Org. Chem.* **1996,** *61*, 4405-4411). It was difficult to chromatographically separate the mixture of **5** and **8** so it was first 5'-desilylated with TBAF and then fractionated using flash silica gel column chromatography. In a typical procedure **7** (7 g, 13 mmol) and Ph₃P (5.25 g. 20 mmol) are dissolved in THF and the mixture is heated to reflux. DEAD (3.15 ml, 20 mmol) is than added to the refluxing mixture and heating continued for 1 hour. Solvent is removed *in vacuo* and the residue purified by flash column chromatography using 9-11% gradient of ethyl acetate in hexanes for elution. 5.6 g, 83% of the mixture of **5** and **8** was obtained after removal of solvents (see Figure 8B). The above mixture is dissolved in THF (75 ml) and treated with 1 M TBAF in THF (22 ml, 2 eq) for 1 hour. It is than concentrated *in vacuo* and chromatographed on the column of silica gei using 15-70% gradient of ethyl acetate in hexanes for elution. α-Anomer 10 eluted first (0.56 g, 19%), followed by β-anomer **9** (1.6 g, 54%). *Lyxo* derivative **11** eluted last (0.55 g, 19%). β-anomer_9 was obtained in 45% yield in two steps from **7**, while α-anomer **10** and *lyxo* derivative **11** were both obtained in 16% yield. **9** was converted into free 2-fluoro nucleoside **12** (mp 134-135 °C, from THF) by boiling in 80% acetic acid and then converted into 2-(benzyloxy) derivative with BnOK (¹H NMR (CD₃OD) data for **12**: δ 8.19 (m, 1H, H6), 8.11 (m, 1H, H4), 7.32 (m, 1H, H5), 4.98 (d, J_{1',2'}=5.6, 1H, H1'), 4.05-3.96 (m, 3H, H2',H3',H4'), 3.84 (dd, J_{5',4'}=3.0, J_{5',5'}=12.0, 1H, H5'), 3.73 (dd, J _{5",4}=4.6, J _{5",5'}=12.0, 1H, H5"). Catalytic hydrogenolysis of benzyl group (H₂, Pd-C) simultaneously cleaved C1'-O4' bond. Debenzylation was carried out successfully with trimethylsilyl iodode (TMSI) to afford 3-(β-D-ribofuranosyl)pyridin-2-one (**13**) in 83% yield as a syrup [UV(MeOH) λₘₐₓ 302 nm]. α-Anomer **10** was deprotected in the same manner as **9** to give **14** (mp 173-174 °C, from CH₂Cl₂; ¹H NMR (CD₃OD) data for **14**: δ 8.12-8.06 (m, 2H, H6,H4), 7.31 (m,1H, H5), 5.26 (d, J_{1',2'}=2.8, 1H, H1'), 4.35-4.25 (m, 2H, H2',H3'). 4.04 (m, 1H, H4'), 3.88 (dd, J_{5',4'}=2.6, J_{5',5"}=11.8, 1H, H5'), 3.68 (dd, J_{5",4'}=4.6, J_{5",5}=11.8, 1H, H5"). An attempt to displace fluorine in 14 with benzylate yielded the anhydro derivative 15 in a quantitative yield [mp 195-196 °C, UV(MeOH) λₘₐₓ 286 nm; ¹H NMR (CD₃)₂CO data for **15**: δ 8.10 (dd, J_{6,5}=5.2, J_{6,4}=1.6, 1H, H6), 7.80 (dd, J_{4,5}=7.3, J_{4,6}=1.6, 1H, H4,). 6.95 (dd, J_{5,6}=5.2, J_{5,4}=7.3, 1H, H5), 5.59 (d, J_{1',2'}=6.0, 1H, H1'), 5.36 (d, J_{OH,3'}=7.2, 1H, 3'OH), 5.02 (dd, J_{2',1'}=6.0, J_{2',3'}=5.2, 1H, H2'), 4.64 (t, J_{5',OH}=5.4, 1H, OH5'), 3.97 (m, 1H, H3'), 3.62 (dq, J_{5',4'}=2.2, J_{5',5'}=12.2, 1H, H5'), 3.39 (m, 1H, H5"), 3.22 (m, 1H, H4')]. This unexpected cyclization can be explained by an intramolecular nucleophilic displacement of fluorine by an adjacent 2'-hydroxyl. The structure of **11** was confirmed by NOE experiments in which mutual enhancement was observed between H4' and H3' (6% NOE) and between H4' and H4 (5% NOE).

Applicant has now described useful methods for the synthesis of larger guantities of β-anomers of C-nucleosides **12** an **13** as starting materials for the preparation of building blocks for oligonucleotide synthesis. These analogs are also interesting as potential antiviral and/or anticancer agents.

### Example 4: Synthesis of 1-(5-O-Dimethoxytrityl-2-O-tert-butyldimethylsilyl-3-O-2-cyanoethyl-N,N-diisopropylaminophosphoramidite-β-D-Ribofuranosyl)-1.4-dihydro-pyrimidine-4-one (7)

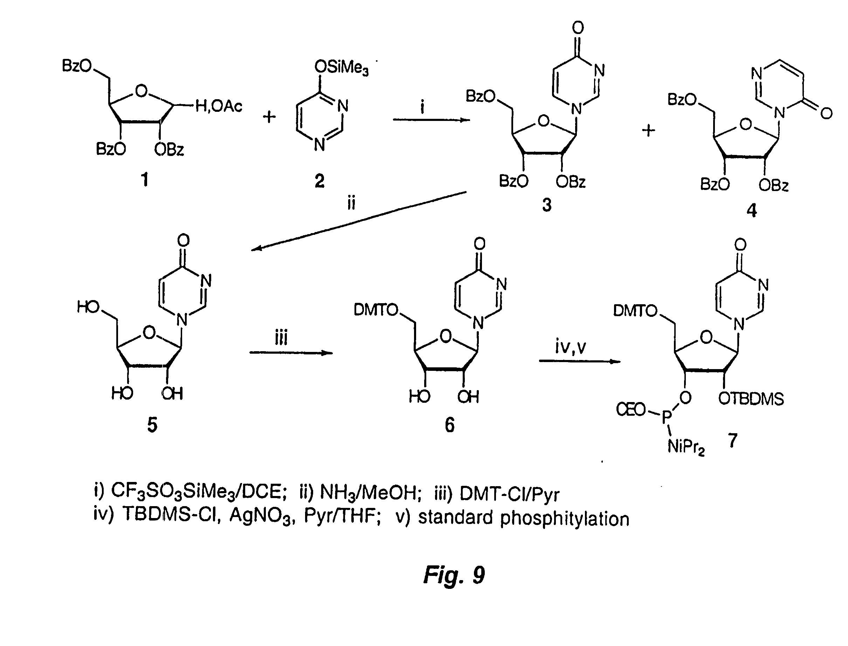

Referring to Figure 9, 1-(2,3,5-Tri-O-benzoyl-β-D-ribofuranosyl)-1,4-dihydropyrimidine-4-one (**3**) was synthesized in accordance with Niedballa *et al., J.Org.Chem* 1974, *39*, 3668-3671. 1-(β-D-Ribofuranosyl)-1,4-dihydropyrimidine-4-one (**5**) was prepared by standard NaOMe/MeOH deprotection of derivative **3** with nearly quantitative yield. 1-(5-O-Dimethoxytrityl-β-D-Ribofuranosyl)-1,4-dihydropyrimidine-4-one (**6**) was prepared by standard dimethoxytritylation of **5** in pyridine (rt., overnight) resulting in a yield of 83%. 1-(5-O-Dimethoxytrityl-2-O-tert-butyldimethylsilyl-β-D-Ribofuranosyl)-1,4-dihydropyrimidine-4-one was prepared using standard silylation procedure (Hakimelahi *et al., Can. J. Chem.* 1982, *60*, 1106-1113). 1-[5-O-Dimethoxytrityl-2-O-tert-butyldimethylsilyl-3-O-2cyanoethyl-N,N-di-isopropylaminophosphoramidite-β-D-Ribofuranosyl]-1,4-dihydro-pyri-midine-4-one (**7**) was prepared by phosphitylation using standard phosphitylation procedure (Tuschl *et al., Biochemistry* 1993, *32*, 11658-11668).

### Example 5: Synthesis of 1-(5-O-Dimethoxytrityl-2-O-tert-butyldimethylsilyl-3-O-2-cyanoethyl-N,N-diisopropylaminophosphoramidite-β-D-Ribofuranosyl)-1,4-dihyrdro-pyrimidine-2-one (6)

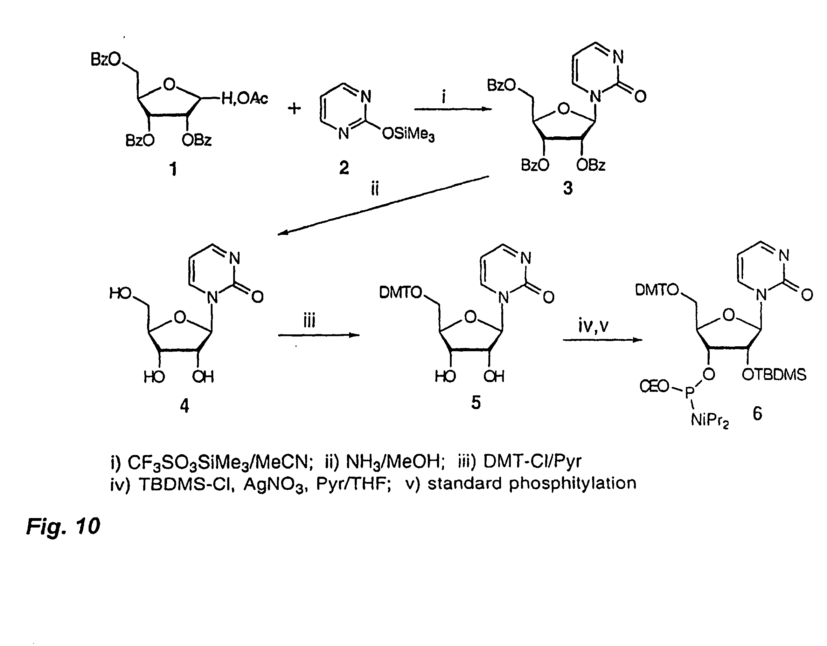

Referring to Figure 10, compound **6** was prepared as described in Murray *et al., Biochem.J.* **1995**, *311*, 487-494.

### Example 6: Synthesis of 5'-O-Dimethoxytrityl-2'-O-tert-Butyldimethylsilyl-O⁴⁻Diphenylcarbamoyl-3-Deaza Uridine 3'-(2-Cyanoethyl N,N-diisopropyl phosphoramidite) (5)

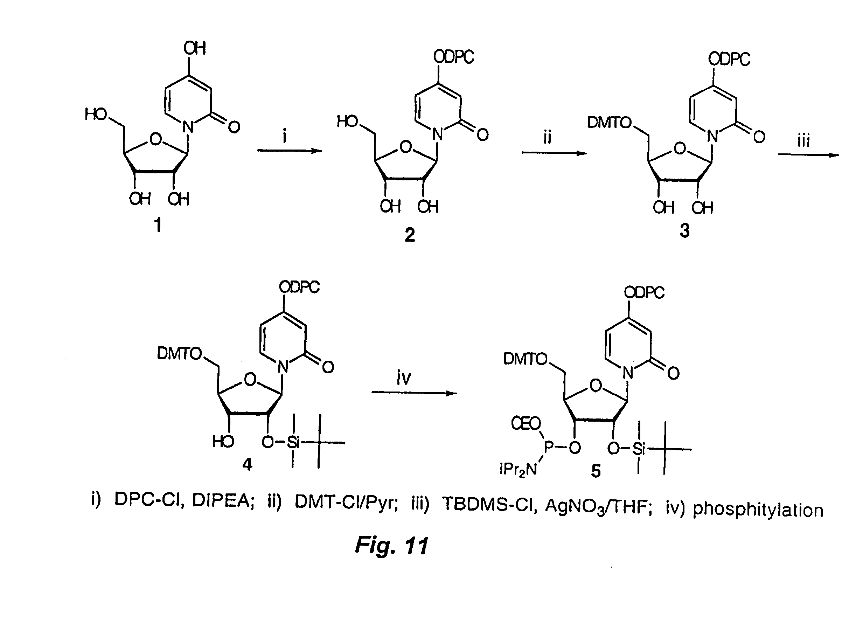

Referring to Figure 11, compound (**5**) was prepared as described in U.S. Patent No. 5,134,066, except for dimethoxytritylation of O⁴⁻Diphenylcarbamoyl-3-deaza-uridine (**2**). The procedure disclosed in the above-mentioned patent afforded desired 5'-*O*-Dimethoxytrityl-O⁴⁻Diphenylcarbamoyl-3-deaza-uridine (**3**) in only 10% yield. Therefore the following procedure for dimethoxytritylation of **2** was developed.

To the solution of compound 2 (1.95g, 4.45 mmol) in dichloromethane (60 mL), symm-collidine (1.53 mL, 11.6 mmol) was added followed by the addition of silver nitrate (0.99g, 5.8 mmol). After stirring at room temperature for 10 min, dimethoxytrityl chloride was added and the reaction mixture was stirred for additional 1h. Then it was quenched with MeOH (15 mL) and evaporated to dryness. The residue was dissolved in dichloromethane, washed with saturated aq sodium bicarbonate and brine. Organic layer was dried over sodium sulfate and the solvent removed in vacuum. The oily residue was purified by flash chromatography on silica using gradient of EtOAc in Hexanes (30% to 50%) as an eluent to give 3g (88.8%) of the derivative **3**.

### Example 7: Synthesis of 2-O-t-Butyldimethylsilyl-5-O-Dimethoxytrityl-3-O-(2-Cyanoethyl-N,N-diisopropylphosphoramidite)-β-D-ribofuranosylindole

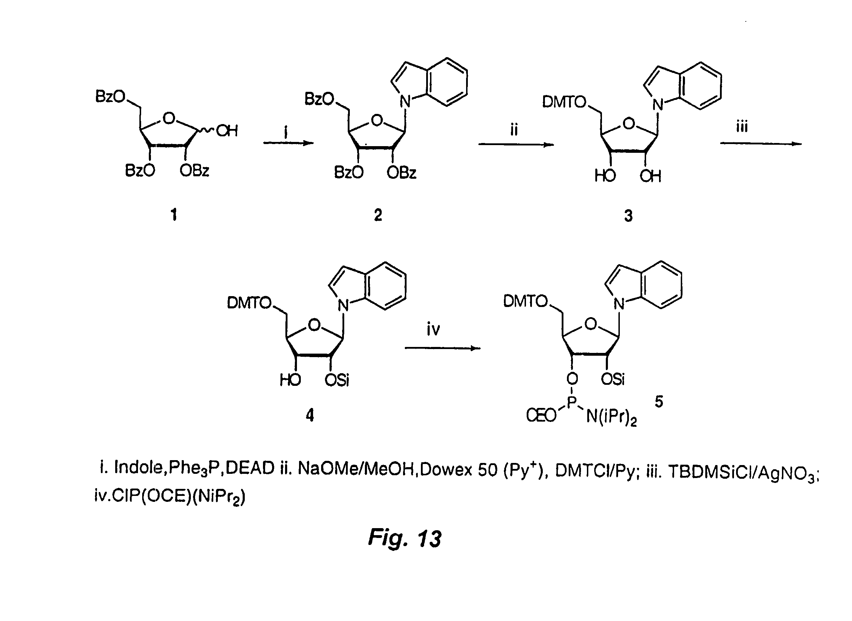

Referring to Figure 13, 1-(2,3,5-Tri-O-benzoyl-β-D-ribofuranosyl) indole (**2**) was synthesized from indole and 2,3,5-tri-O-benzoyl-β-D-ribofuranose (1) according to the procedure of Szarek *et al., Chem.Comm.* **1975,** *648-649*)*.* 5-O-dimethoxytrityl β-D-ribofuranozylindole (**3**) was prepared from intermediate **2** by standard deprotection(NaOMe/MeOH) followed by tritylation (DMTCI/Py). 2-*O*-*t*-Butyidimethylsilyl-5-*O-*dimethoxytrityl β-D-ribofuranozylindole (**4**) was prepared from intermediate **3** by standard silylation (32% yield). 2-*O*-*t*-Butyldimethylsilyl-5-*O-*dimethoxytrityl-3-*O*-(2-cyanoethyl-*N,N*-diisopropylphosphoramidite)-_β-D-ribofuranozylindole (**5**) was prepared from intermediate **4** by 3'-O-phosphitylation (85% yield).

### Example 8: Synthesis of 2-O-t-Butyldimethylsilyl-5-O-Dimethoxytrityl-3-O-(2-Cyanoethyl-N,N-diisopropylphosphoramidite)-β-D-ribofuranosyl benzimidazole

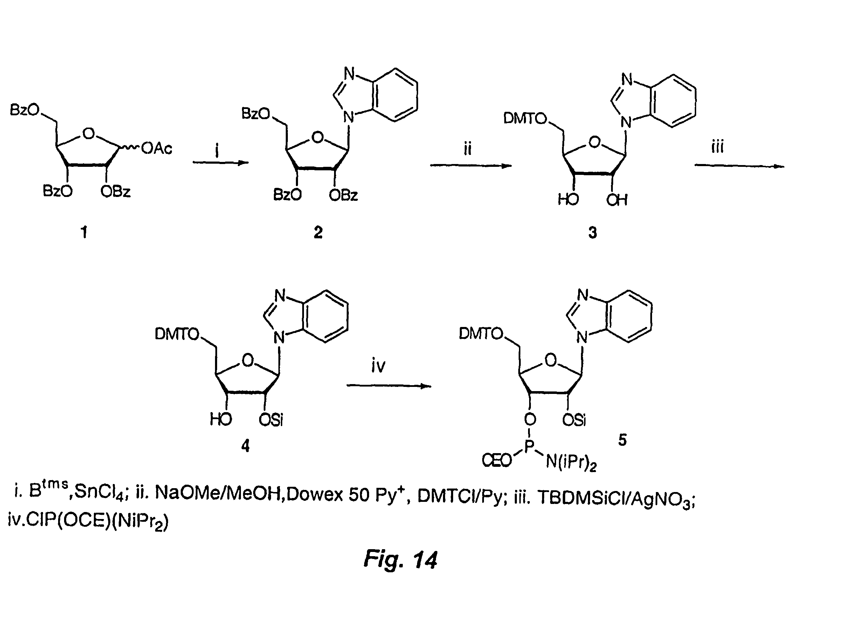

Referring to Figure 14, 1-(2,3,5-Tri-O-benzoyl-β-D-ribofuranosyl) benzimidazole (**2**) was synthesized from benzimidazole and 1-O-acetyl-2,3,5-tri-O-benzoyl-β-D-ribofuranose (**1**) according to the procedure of Kazimierczuk *et al., Naturforsch* **.1980,** *35c* , 30-35. 5'-*O*-dimethoxytrityl β-D-ribofuranozyl benzimidazole (**3**) was prepared from intermediate **2** by standard deprotection (NaOMe/MeOH) followed by tritylation (DMTCI/Py). 2'-*O*-*t*-Butyldimethylsilyl-5'-*O*-dimethoxytritylβ-D-ribofuranozylbenzimidazole (**4**) was prepared from intermediate **3** by standard silylation (32% yield). 2'-*O-t*-Butyldimethyfsilyl-5'-*O*-dimethoxytrityl-3-*O*-(2-cyanoethyl-*N,N*-diisopropyl phosphoramidite)-β-D-ribofuranozyl benzimidazole (**5**) was prepared from intermediate **4** by 3'-O-phosphitylation (85% yield).

### Example 9: Synthesis of 3-(β-D-Ribofuranosyl)-Pyridine-2-(4-Nitrophenylethyl)-One and 5-(β-D-Ribofuranosyl)-Pyridine-2-(4-Nitrophenylethyl)-One Phosphoramidite.

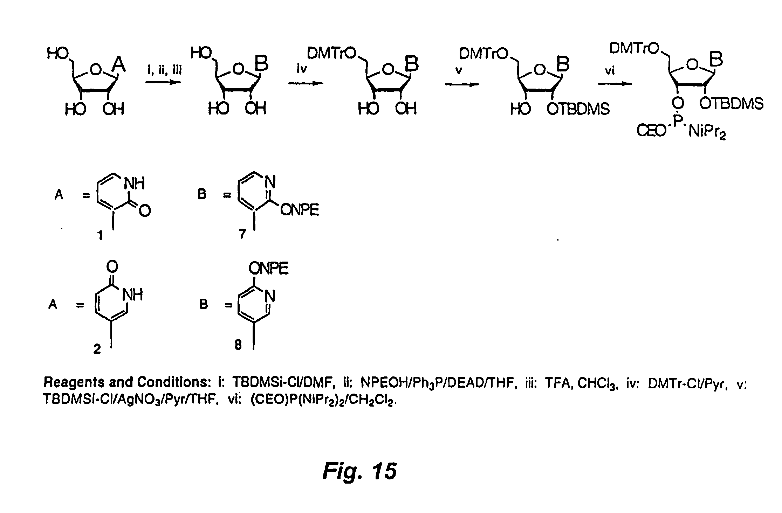

Referring to Figure 15, treatment of 3-(β-D-Ribofuranosyl)-pyridin-2-one (1 in Figure 6) with TBDMSi-Cl/DMF, NPEOH/Ph3P/DEAD/THF, and TEA. CHCl3, will yield 3-(β-D-Ribofuranosyl)-pyridin-2-(4-nitrophenylethyl)-one **(7).** Treatment of 5-(β-D-Ribofuranosyl)-pyridin-2-one **(2** in Figure 6) with TBDMSi-CUDMF, NPEOH/Ph3P/DEAD/THF, and TFA, CHCl3, will yield 5-(β-D-Ribofuranosyl)-pyridin-2-(4-nitrophenylethyl)-one (**8**).

Both compounds **7** and **8** is readily converted into phosphoramidites using the standard protocols described above.

Phosphoramidites of the novel anologs of the instant invention are incorporated into polynucleotides using the method of synthesis, deprotection, purification and testing previously described (Wincott *et al.,* 1995 *supra).*

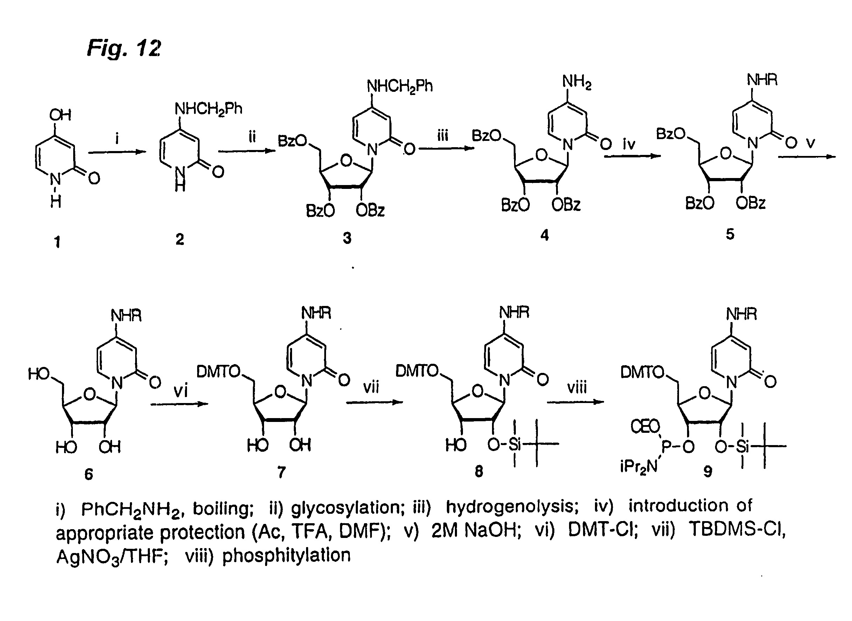

### Example 10: 4-Benzylamino-1H-pyridine-2-one or N⁴-benzyl-3-deaza cytosine (figure 12)

Title compound was prepared from 4-hydroxy-1H-pyridin-2-one (1) as described in Hung, N.C.; Bisagni, E. *Synthesis,* 1984, 765.

*4-Benzylamino-1-(2,3,5-tri-O-benzoyl-β-D-ribofuranosyl)-1H-pyridin-2-one (**3**) or N*^{*4*}*-benzyl-2',3',5'-tri-O-benzoyl-3-deaza cytidine:* The suspension of **2** (2.8g, 14.0 mmol) in the mixture of hexamethyldisilazane (50mL) and trimethylchlorosilane (5mL) was refluxed for three hours. The resulting clear solution of trimethylsilyl derivative of **2** was evaporated to dryness. To the solution of the resulting clear oil in dry acetonitrile (40 mL), 1-*O*-acetyl-2,3,5-tri-*O*-benzoyl-D-ribofuranose (7.06g, 14.7 mmol) was added and the reaction mixture was cooled to 0°C. To the above stirred solution trimethylsilyl trifluoromethanesulfonate (3.25 mL, 16.8 mmol) was added drop-wise and the reaction mixture was allowed to warm to a room temperature and left overnight. After that the reaction mixture was diluted with dichloromethane washed with saturated sodium bicarbonate. The precipitate of the unreacted **2**, was isolated by filtration. Then layers were separated and organic layer washed with brine and evaporated. The residue was purified by flash chromatography on silica gel to give 5.5g (60%) of the compound **3**.

*4-Amino-1-(2,3,5-tri-O-benzoyl-β-D-ribofuranosyl)-1H-pyridin-2-one (**4**) or 2',3',5'-tri-O-benzoyl-3-deaza cytidine:* Compound **4** is prepared by catalytic hydrogenolysis (Pd/C) of **3** in ethanol at room temperature.

*4-Acetylamino-1-(2,3,5-tri-O-benzoyl-β-D-ribofuranosyl)-1H-pyridin-2-one (**5**):* Compound **5** is prepared by standard acetylation of the derivative **4** with acetic anhydride in pyridine.

*4-Acetylamino-1-(β-D-ribofuranosyl)-1H-pyridin-2-one* (***6***): The title compound is prepared by selective deprotection of sugar moiety of derivative **5** with 2M aq NaOH in pyridine-ethanol mixture at -10°C.

*4-Acetylamino-1-(5-O-dimethoxytrityl-β-D-ribofuranosyl)-1H-pyridin-2-one (**7**)*: compound **7** is prepared using standard protocols described above.

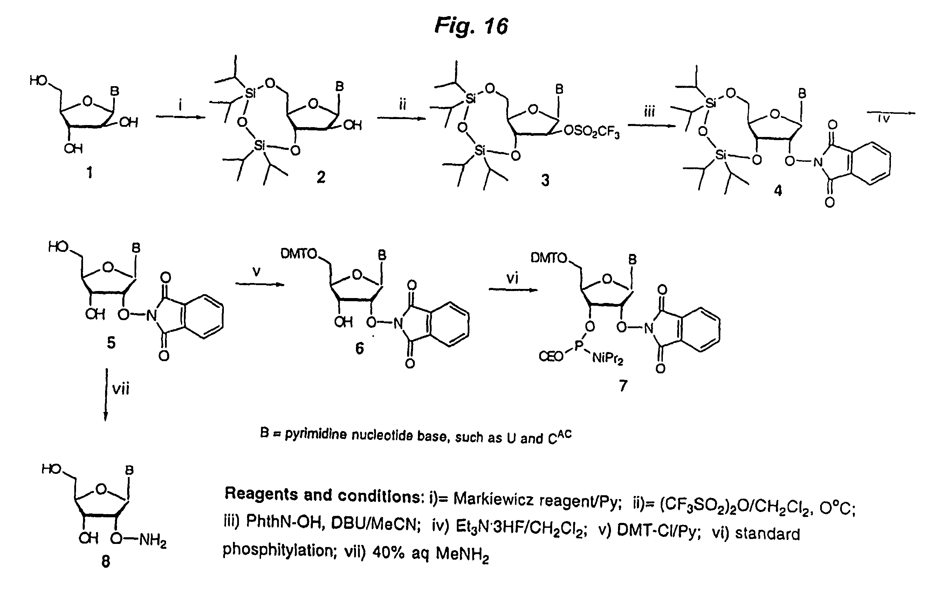

### Example 11: Synthesis of 2'-O-amino pyrimidine nucleoside (8 in Figure 16)

Synthetic methods for preparing 5'-O-NH₂ or 3'-O-NH₂ derivatives of 2'-deoxynucleosides by Mitsunobu inversion are well known in the art. However, applicants attempts to apply this method to the preparation of 2'-O-NH₂-ribonucleosides resulted in very low yields (< 10%) of desired compounds. Applicant has developed a new and highly efficient process for the synthesis of 2'-*O*-NH₂-ribonucleosides and their phosphoramidites.

### 3',5'-O-(tetraisopropyldisiloxane-1,3-di-yl)-1-β-D-arabinofuranosyl-pyrimidine (2)

Referring to Figure 16,1-β-D-arabinofuranosyl-pyrimidine, such as 1-β-D-arabinofuranosyl-uracil (2.44g, 10 mmol) was dried by two co-evaporations with anhydrous pyridine and re-dissolved in the anhydrous pyridine. The above solution was cooled (0°C) and solution of 1,3-dichloro-1,1,3,3-tetraisopropylsiloxane (3.52 mL, 11.0 mmol) in 10 mL of anhydrous dichloroethane was added drop-wise under stirring. The reaction mixture was allowed to warm to room temperature and stirred for additional two hours. The reaction was quenched with MeOH (10 mL) and evaporated to dryness. The residue was dissolved in methylene chloride and washed with saturated NaHCO₃ and brine. The organic layer was evaporated to dryness and coevaporated with toluene to remove traces of pyridine to give 4.8g (98%) of compound **2** which was used without further purification.

### 2'-O-phthalimido-3',5'-O-(tetraisopropyldisiloxane-1,3-di-yl)-uridine(4)

To the ice-cooled solution of 3',5'-O-(tetraisopropyldisiloxane-1,3-di-yl)-1-β-D-arabinofuranosyt-uracil (4g, 8.2 mmol) in dichloromethane trifluoromethane sulfonic anhydride (1.66 mL, 9.86 mmol) was added and the reaction mixture was stirred at -5°C for 30 min. Then it was diluted with dichloromethane and washed with cold 1% aq acetic acid, then with saturated aq sodium bicarbonate and brine. Organic layer was dried over anhydrous sodium sulfate and evaporated to dryness. Resulting derivative **3** was dissolved in anhydrous acetonitrile (70 mL) and N-hydroxyphtalimide (1.74g, 10.66 mmol was added. Solution of DBU (1.6 mL, 10.66 mmol) in acetonitrile (5 mL) was added dropwise to the reaction mixture under vigorous stirring. After 30 min dark orange reaction mixture was diluted with dichloromethane (250 mL) and extracted with saturated aq sodium bicarbonate solution (3x250 mL). Resulted colorless organic layer was washed with brine and evaporated to give 3.6g (70%) of compound **4**.

### 2'-O-phthalimido-uridine (5)

Triethylamine (1.32 mL, 9.5 mmol) and triethylamine trihydrofluoride (1.55 mL, 9.5 mmol) were added simultaneously to the solution of 2'-*O-*phthalimido-3',5'-*O*-(tetraisopropyldisiloxane-1,3-di-yl)-uridine (3g, 4.75 mmol) in dichloromethane. After 1 hour the solvents were removed in vacuo, and remaining residue was dissolved in dichloromethane and evaporated at 35°C. This procedure was repeated 3 times (untill Thin Layer Chromatography showed complete conversion of the starting material). The residue was dissolved in dichloromethane and washed with saturated aq sodium bicarbonate and brine. Organic layer was dried over anhydrous sodium sulfate and evaporated to dryness to give 1.56g (85%) of compound **5**.

### 2'-O-amino uridine (8) was obtained by hydrolysis with 40% aq methylamine and subsequent crystallization from ethanol.

### 5'-O-dimethoxytrityl-2'-O-phthalimido-uridine (6)

The compound 5 (1.5g, 4 mmol) was dried by multiple evaporations with anhydrous pyridine, redissolved in dry pyridine, dimethoxytritylchloride (1.2 eq) was added and the reaction mixture was left under anhydrous conditions overnight. Then it was quenched with methanol (15 ml), evaporated, dissolved in chloroform, washed with saturated aq sodium bicarbonate and brine. Organic layer was dried over sodium sulfate and evaporated. The residue was purified by flash chromatography on silica gel eluting with EtOAc - hexanes mixture (2:3) to give 1.93g (70%) of the corresponding 5'-O-Dimethoxytrityl derivative **6**.

### 5'-O-Dimethoxytrityl-2'-O-phthalimido uridine-3'-(2-Cyanoethyl N,N-diisopropyl) phosphoroamidite (7)

Standard phosphitylation of **6** according to Tuschl *et al., Biochemistry* **1993**, 32, 11658-11668) yielded phosphoramidite **7** in 70% yield.

General Procedure for Phosphitylation: To the ice-cooled stirred solution of protected nucleoside (1 mmol) in dry dichloromethane (20 mL) under argon blanket was added dropwise via syringe the premixed solution of N,N-diisopropylethylamine (2.5eq) and 2-cyanoethyl N'N-diisopropylchlorophosphoramidite (1.2 eq) in dichloromethane (3 mL). Simultaneously via another syringe N-methylimidazole (1 eq) was added and stirring was continued for 2 hours at room temperature. After that the reaction mixture was again ice-cooled and quenched with 15 ml of dry methanol. After 5 min stirring, the mixture was concentrated in vacuo (<40°C) and purified by flash chromatography on silica gel using hexanes-ethylacetate mixture contained 1% triethylamine as an eluent to give corresponding phosphoroamidite as white foam.

Phosphoramidites were incorporated into nucleic add molecules, such as ribozymes and substrates, using the method of synthesis, deprotection, purification and testing previously described (Wincott *et al.,* 1995 *supra).* The average stepwise coupling yields were ~98 %.

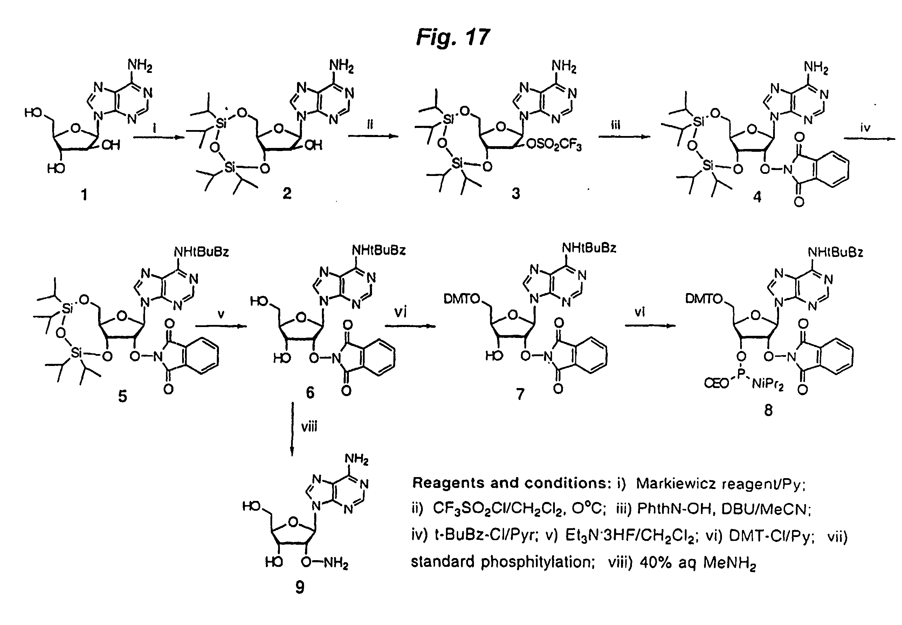

### Example 12: Synthesis of 2'-O-phthalimido-3',5'-O-(tetraisopropyldisiloxane-1,3-di-yl)-N⁶-t-butybenzoyl adenosine (5 in Figure 17) and 2'-O-amino adenosine nucleoside (9 in Figure 17)

Referring to Figure 17, 9-β-D-arabinofuranosyl adenine (12 mmol) was silylated with 1,3-dichloro-1,1,3,3-tetraisopropylsitoxane (4.2 mL, 13.2 mmol) as described for the ara-uridine derivative. The cold solution (-10°C) of the product in anhydrous dichloromethane was treated with trifluoromethanesulfochloride (1.53 mL, 14.4 mmol) for 20 min. Resulted solution was diluted with anhydrous dichloromethane and washed with cold (0°C) 1% aq acetic acid, then saturated aq NaHCO₃ and brine. Organic layer was dried over sodium sulfate and evaporated to dryness to give derivative **2.** The residue was dissolved in anhydrous acetonitrile and N-hydroxyphthalimide (2.54g, 15.6 mmol) was added. Solution of DBU (2.33 mL 15.6 mmol) in anhydrous acetonitrile was added to the resulted reaction mixture under vigorous stirring. After 30 min dark orange-brown reaction mixture was worked-up as described for uridine derivative. Resulted 2'-O-phthalimido derivative **4** was dissolved in anhydrous pyridine, 4-tert-butylbenzoyl chloride was added and reaction mixture was left overnight at room temperature. After that it was quenched with methanol (15 mL), solvents removed in vacuo and the residue dissolved in toluene and evaporated to dryness. Resulted oil was dissolved in dichloromethane, washed with saturated aq NaHCO₃ and brine, dried over sodium sulfate and evaporated to dryness. The residue was purified by flash chromatography on silica, using EtOAc-Hexanes (1:2) mixture as an eluent to give 3.5 g (35% on ara-A) of the fully protected synthon **5**.

### 2'-O-phthalimido-N⁸-t-butylbenzoyl adenosine (6)

Markiewicz-group deprotection was performed analogously to the corresponding uridine derivative. Yield 86%.

*2'-O-amino adenosine* **(9)** was obtained by hydrolysis with 40% aq methylamine and subsequent crystallization from ethanol.

### 5'-O-Dimethoxytrityl-2'-O-phthalimido-N⁶-t-butylbenzoyladenosine(7)

Standard dimethoxytritylation of 2'-*O*-phthalimido-N⁶-t-butylbenzoyl adenosine afforded compound 7 with 75% yield.

### 5'-O-Dimethoxytrityl-2'-O-phthalimido-N⁶-t-butylbenzoly adenosine 3'-(2-Cyanoethyl N,N-diisopropyl) phosphoroamidite (8)

Standard phosphitylation of according Tuschl *et al., supra* yielded phosphoramidite **8** in 70% yield.

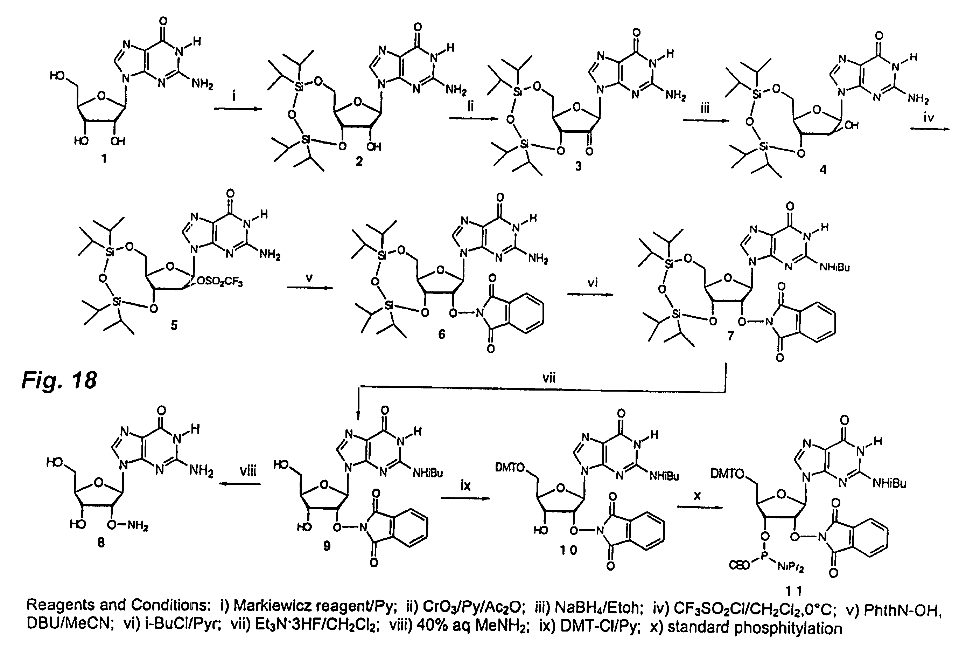

### Example 13: Synthesis of 2'-O-phthalimido-3',5'-O-(tetraisopropyldisiloxane-1,3-di-yl)-N⁶-t-butylbenzoyl Guanosine (11 in Figure 18) and 2'-O-amino Guanosine nucleoside (8 in Figure 18)

Referring to Figure 18, compound **4** is synthesized starting from compound **1,** using the process described in Hansske *et al.,* 1984, *Tetrahedron* 40, 125, incorporated by reference herein. Compounds **8** and **9** can then be synthesized from compound **4** using the procedures described in Figure 7.

### Example 14: RNA cleavage reaction catalyzed by ribozymes substituted with 2'-O-amino modifications

### RNA cleavage assay in vitro:

Substrate RNA is 5' end-labeled using [γ-³²P] ATP and T4 polynucleotide kinase (US Biochemicals). Cleavage reactions are carried out under ribozyme "excess" conditions. Trace amount (≤ 1 nM) of 5' end-labeled substrate and 40 nM unlabeled ribozyme are denatured and renatured separately by heating to 90°C for 2 min and snap-cooling on ice for 10 -15 min. The ribozyme and substrate are incubated, separately, at 37°C for 10 min in a buffer containing 50 mM Tris-HCl and 10 mM MgCl₂. The reaction is initiated by mixing the ribozyme and substrate solutions and incubating at 37°C. Aliquots of 5 µl are taken at regular intervals of time and the reaction is quenched by mixing with equal volume of 2X formamide stop mix. The samples are resolved on 20 % denaturing polyacrylamide gels. The results are quantified and percentage of target RNA cleaved is plotted as a function of time.

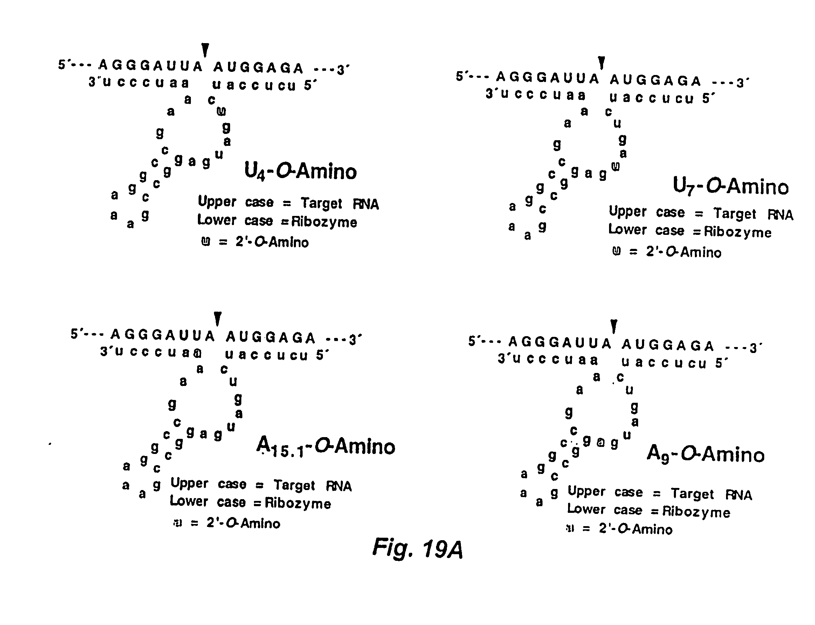

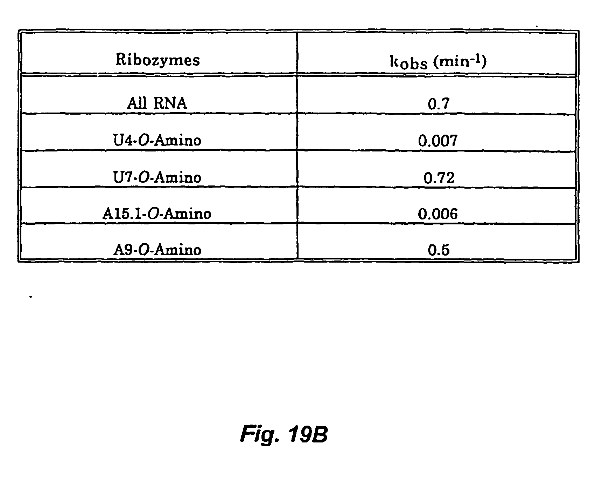

Referring to Figure 19A, hammerhead ribozymes with 2'-*O*-amino-substitutions at position 4, 7, 9 or 15.1 were synthesized as described above. These ribozymes were assayed for their ability to cleave target RNA. As shown in Figure 19B, all the ribozymes shown in Figure 19A were capable of catalyzing cleavage of target RNA.

### Applications

Various ligands can be attached to oligonucleotides using the nucleosides or nucleotides of the present invention for the purposes of cellular delivery, nuclease resistance, cellular trafficking and localization, chemical ligation of oligonucleotide fragments. Incorporation of one or more nucleosides or nucleotides of the invention into a ribozyme may increase its effectiveness. The nucleosides /nucleotides of the invention can also be used as potential antiviral agents.

### Ribozyme Engineering

Sequence, chemical and structural variants nucleic acid catalysts can be engineered using the techniques shown above and known in the art to cleave a separate target RNA or DNA in *trans* (Zaug *et al.,* 1986, *Nature,* 324, 429; Ruffner *et al.,* 1990, *Biochem.,* 29, 10695; Beaudry *et al.,* 1990, *Biochem.,* 29, 6534; McCall *et al.,* 1992, *Proc. Natl. Acad. Sci., USA.,* 89, 5710; Long *et al.,* 1994, Supra; Hendry *et al.,* 1994, *BBA* 1219, 405; Benseler *et al.,* 1993, *JACS,* 115, 8483; Thompson *et al.,* 1996, *Nucl. Acids Res.,* 24, 4401;Michels *et al.,* 1995, *Biochem.,* 34, 2965; Been *et al.,* 1992, *Biochem.,* 31, 11843; Guo *et al.,* 1995, *EMBO. J.,* 14, 368; Pan *et al.,* 1994, *Biochem.,* 33, 9561; Cech, 1992, *Curr. Op. Struc. Bio.,* 2, 605; Sugiyama *et al.,* 1996, *FEBS Lett.,* 392, 215; Beigelman *et al.,* 1994, *Bioorg. Med. Chem.,* 4, 1715).

### Diagnostic uses

Ribozymes of this invention may be used as diagnostic tools to examine genetic drift and mutations within diseased cells or to detect the presence of a specific_RNA in a cell. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules). Other in vitro uses of ribozymes of this invention are well known in the art, and include detection of the presence of mRNAs associated with related condition. Such RNA is detected by determining the presence of a cleavage product after treatment with a ribozyme using standard methodology.

In a specific example, ribozymes which can cleave only wild-type or mutant forms of the target RNA are used for the assay. The first ribozyme is used to identify wild-type RNA present in the sample and the second ribozyme will be used to identify mutant RNA in the sample. As reaction controls, synthetic substrates of both wild-type and mutant RNA will be deaved by both ribozymes to demonstrate the relative ribozyme efficiencies in the reactions and the absence of cleavage of the "non-targeted" RNA species. The cleavage products from the synthetic substrates will also serve to generate size markers for the analysis of wild-type and mutant RNAs in the sample population. Thus each analysis will require two ribozymes, two substrates and one unknown sample which will be combined into six reactions. The presence of cleavage products will be determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype is adequate to establish risk. If probes of comparable specific activity are used for both transcripts, then a qualitative comparison of RNA levels will be adequate and will decrease the cost of the initial diagnosis. Higher mutant form to wild-type ratios will be correlated with higher risk whether RNA levels are compared qualitatively or quantitatively.

**TABLE 1**

| **Characteristics of naturally occurring ribozymes** | |
|---|---|
| **Group I Introns** | |
| • | Size: ~150 to >1000 nucleotides. |
| • | Requires a U in the target sequence immediately 5' of the cleavage site. |
| • | Binds 4-6 nucleotides at the 5'-side of the cleavage site. |
| • | Reaction mechanism: attack by the 3'-OH of guanosine to generate cleavage products with 3'-OH and 5' guanosine. |
| • | Additional protein cofactors required in some cases to help folding and maintenance of the active structure [¹]. |
| • | Over 300 known members of this class. Found as an intervening sequence in *Tetrahymena thermophila* rRNA, fungal mitochondria, chloroplasts, phage T4, blue-green algae, and others. |
| • | Major structural features largely established through phylogenetic comparisons, mutagenesis, and biochemical studies [^{2,3}]. |
| • | Complete kinetic framework established for one ribozyme [^{4,5,6,7}]. |
| • | Studies of ribozyme folding and substrate docking underway [^{8,9,10}]. |
| • | Chemical modification investigation of important residues well established [^{11,12}]. |
| • | The small (4-6 nt) binding site may make this ribozyme too non-specific for targeted RNA cleavage, however, the Tetrahymena group I intron has been used to repair a "defective" β-galactosidase message by the litigation of new β-galactosidase sequences onto the defective message [¹³]. |

| **RNAse P RNA (M1 RNA)** | |
|---|---|
| • | Size: ∼290 to 400 nucleotides. |
| • | RNA portion of a ubiquitous ribonucleoprotein enzyme. |
| • | Cleaves tRNA precursors to form mature tRNA [¹⁴]. |
| • | Reaction mechanism: possible attach by M²⁺-OH to generate cleavage products with 3'-OH and 5'-phosphate. |
| • | RNAse P is found throughout the prokaryotes and eukaryotes. The RNA subunit has been sequenced from bacteria, yeast, rodents and primates. |
| • | Recruitment of endogenous RNAse P for therapeutic applications is possible through hybridization of an External Guide Sequence (EGS) to the target RNA [^{15,16}]. |
| • | Important phosphate and 2' OH contacts recently identified [^{17,18}]. |

| **Group II Introns** | |
|---|---|
| • | Size: >1000 nucleotides. |
| • | Trans cleavage of target RNAs recently demonstrated [^{19,20}] |
| • | Sequence requirements not fully determined. |
| • | Reaction mechanism: 2'-OH of an internal adenosine generates cleavage products with 3'-OH and a "lariat" RNA containing a 3'-5' and a 2'-5' branch point. |
| • | Only natural ribozyme with demonstrated participation in DNA cleavage [^{21,22}] in addition to RNA cleavage and ligation. |
| • | Major structural features largely established through phylogenetic comparisons [²³]. |
| • | Important 2' OH contacts beginning to be identified [²⁴]. |
| • | Kinetic framework under development [²⁵]. |

| **Neurospora VS RNA** | |
|---|---|
| • | Size: ∼144 nucleotides. |
| • | Trans cleavage of hairpin target RNAs recently demonstrated [²⁶]. |
| • | Sequence requirements not fully determined. |
| • | Reaction mechanism: attack by 2'-OH 5' to the scissile bond to generate cleavage products with 2', 3'-cyclic phosphate and 5'-OH ends. |
| • | Binding sites and structural requirements not fully determined. |
| • | Only 1 known member of this class. Found in Neurospora VS RNA. |

| **Hammerhead Ribozyme** (see text for references) | |
|---|---|
| • | Size: ~13 to 40 nucleotides. |
| • | Requires the target sequence UH immediately 5' of the cleavage site. |
| • | Binds a variable number nucleotides on both sides of the cleavage site. |
| • | Reaction mechanism: attack by 2'-OH 5' to the scissile bond to generate cleavage products with 2', 3'-cydic phosphate and 5'-OH ends. |
| • | 14 known members of this class. Found in a number of plant pathogens (virusoids) that use RNA as the infectious agent. |
| • | Essential structure features largely defined, including 2 crystal structures []. |
| • | Minimal ligation activity demonstrated (for engineering through *in vitro* selection) []. |
| • | Complete kinetic framework established for two or mor ribozymes []. |
| • | Chemical modification investigation of important residues well established []. |

| **Hairpin Ribozyme** | |
|---|---|
| • | Size: ∼50 nucleotides. |
| • | Requires the target sequence GUC immediately 3' of the cleavage site. |
| • | Binds 4-6 nucleotides at the 5' side of the cleavage site and a variable number to the 3'-side of the cleavage site. |
| • | Reaction mechanism: attack by 2'-OH 5' to the scissile bond to generate cleavage produces with 2', 3'-cyclic phosphate and 5'-OH ends. |
| • | 3 known members of this class. Found in three plant pathogen (satellite RNAs of the tobacco ringspot virus, arabis mosaic virus and chicory yellow mottle virus) which uses RNA as the infectious agent. |
| • | Essential structural features largely defined [^{27,28, 29, 30}]. |
| • | Ligation activity (in addition to cleavage activity) makes ribozyme amenable to engineering through *in vitro* selection [³¹] |
| • | Complete kinetic framework established for one ribozyme [³²]. |
| • | Chemical modification investigation of important residues begun [^{33, 34}]. |

| **Hepatitis Delta Virus (HDV) Ribozyme** | |
|---|---|
| • | Size: ∼60 nucleotides. |
| • | Trans cleavage of target RNAs demonstrated [³⁵]. |
| • | Binding sites and structural requirements not fully determined, although no sequences 5' of cleavage site are required. Folded ribozyme contains a pseudoknot structure [³⁸]. |
| • | Reaction mechanism: attack by 2'-OH 5' to the scissile bond to generate cleavage products with 2', 3'-cydic phosphate and 5'-OH ends. |
| • | Only 2 known members of this class. Found in human HDV. |
| • | Circular form of HDV is active and shows increased nuclease stability [³⁷]. |
| ¹. Mohr, G.; Caprara, M.G.; Guo, Q.; Lambowitz, A.M. Nature, 370, 147-150 (1994). | |
| ². Michel, Francois; Westhof, Eric. Slippery substrates. Nat Struct. Biol. (1994). 1(1), 5-7. | |
| ³. Lisacek, Frederique; Diza, Yolande; Michel, Francois. Automatic identification of group I intron cores in genomic DNA sequences. J. Mol. Bio. (1994), 235(4), 1206-17. | |
| ⁴. Herschlag, Daniel; Cech, Thomas R., Catalysis of RNA cleavage by the Tetrahymena thermophila ribozyme. 1. Kinetic description of the reaction of an RNA substrate complementary to the active site. Biochemistry (1990), 29(44), 10159-71. | |
| ⁵. Herschlag, Daniel; Cech, Thomas R., Catalysis of RNA cleavage by the Tetrahymena thermophila ribozyme. Kinetic description of the reaction of an RNA substrate that forms a mismatch at the active site. Biochemistry (1990), 29(44), 10172-80. | |
| ⁶. Knitt, Deborah S.; Herschlag, Daniel. pH Dependencies of the Tetrahymena Ribozyme Reveal an Unconventional Origin of an Apparent pKa. Biochemistry (1996), 35(5),1560-70. | |
| ⁷. Bevilacqua, Philip C.; Sugimoto, Naoki; Turner, Douglas H., A mechanistic framework for the second step of splicing catalyzed by the Tetrahymena ribozyme. Biochemistry (1996), 35(2), 648-58. | |
| ⁸. Li, Yi; Bevilacqua, Philip C.; Mathews, David; Turner, Douglas H., Thermodynamic and activation parameters for binding a pyrene-labeled substrate by the Tetrahymena ribozyme: docking is not diffusion-controlled and is driven by a favorable entropy change. Biochemistry (1995), 34(44), 14394-9. | |
| ⁹. Banerjee, Aloke Raj; Turner, Douglas H., The time dependence of chemical modification reveals slow steps in the folding of a group I ribozyme. Biochemistry (1995), 34(19), 6504-12. | |
| ¹⁰. Zarrinkar, Patrick P.; Williamson, James R.. The P9.1-P9.2 peripheral extension helps guide folding of the Tetrahymena ribozyme. Nucleic Acids Res. (1996), 24(5), 854-8. | |
| ¹¹. Strobel, Scott A.; Cech, Thomas R., Minor groove recognition of the conserved G.cntdot.U pair at the Tetrahymena ribozyme reaction site. Science (Washington, D.C) (1995), 267(5198), 675-9. | |
| ¹². Strobel, Scott A.; Cech, Thomas R., Exocyclic Amine of the Conserved G.cntdot.U Pair at the Cleavage Site of the Tetrahymena Ribozyme Contributes to 5'-Splice Site Selection and Transition State Stabilization. Biochemistry (1996), 35(4), 1201-11. | |
| ¹³. Sullenger, Bruce A.; Cech, Thomas R., Ribozyme-mediated repair of defective mRNA by targeted trans-splicing. Nature (London) (1994), 371(6498), 619-22. | |
| ¹⁴. Robertson, H.D.; Altman, S.; Smith, J.D. J. Biol. Chem., 247, 5243-5251 (1972). | |
| ¹⁵. Forster, Anthony C.; Altman, Sidney. External Guide Sequences for an RNA enzyme. Science (Washington, D.C., 1993-) (1990), 249(4970), 783-6. | |
| ¹⁶. Yuan, Y.; Hwang, E.S.; Altman, S. Targeted cleavage of mRNA by human Rnase P. Proc. Natl. Acad. Sci. USA (1992) 89, 8006-10. | |
| ¹⁷. Harris, Michael E.; Pace, Norman R.. Identification of phosphates involved in catalysis by the ribozyme Rnase P RNA. RNA (1995), 1 (2), 210-18. | |
| ¹⁸. Pan, Tao; Loria, Andrew; Zhong, Kun. Probing of tertiary interactions in RNA: 2'-hydroxyl-base contacts between the Rnase P RNA and pre-tRNA. Proc.Natl.Acad.Sci. U.S.A. (1995), 92(26), 12510-14. | |
| ¹⁹. Pyle, Anna Marie; Green, Justin B.. Building a Kinetic Framework for Group II Intron Ribozyme Activity: Quantitation of Interdomain Binding and Reaction Rate. Biochemistry (1994), 33(9), 2716-25. | |
| ²⁰. Michaels, William J. Jr.; Pyle, Anna Marie. Conversion of a Group II Intron into a New Multiple-Tumover Ribozyme and Selectively Cleaves Oligonucleotides: Elucidation of Reaction Mechanism and Structure/Function Relationships. Biochemistry (1995), 34(9), 2965-77. | |
| ²¹. Zimmerly, Steven; Guo, Huatao; Eskes, Robert; Yang, Jian; Perlman, J.; Philip S.; Lambowitz, Alan M.. A group II intron RNA is a catalytic component of a DNA endonuclease involved in intron mobility. Cell (Cambridge, Mass.) (1995), 83(4), 529-38. | |
| ²². Griffin, Edmund A., Jr.; Qin, Zhifeng; Michels, Williams J., Jr.; Pyle, Anna Marie. Group II intron RNA is a catalytic component of a DNA endonuclease involved in intron mobility. Cell (Cambridge, Mass.) (1995), 2(11), 761-70. | |
| ²³. Michel, Francois; Ferat, Jean Luc. Structure and activities of group II introns. Annu. Rev. Biochem. (1995), 64,435-61. | |
| ²⁴. Abramovitz, Dana L; Friedman, Richard A.; Pyle, Anna Marie. Catalytic role of 2'-hydroxyl groups within a group II intron active site. Science (Washington, D.C.) (1996), 271(5254), 1410-13. | |
| ²⁵. Daniels, Danette L.; Michels, William J., Jr.; Pyle, Anna Marie. Two competing pathways for self-splicing by group 11 introns: a quantitative analysis of in vitro reaction rates and products. J. Mol. Biol. (1996), 256(1), 31-49. | |
| ²⁶. Guo, Hans C.T.; Collins, Richard A.. Efficient trans-cleavage of a stem-loop RNA substrate by a ribozyme derived from Neurospora VS RNA. EMBO J. (1995), 14(2), 368-76. | |
| ²⁷. Hampel, Arnold; Tritz, Richard; Hicks, Margaret; Cruz, Phillip. 'Hairpin' catalytic RNA model: evidence for helixes and sequence requirement for substrate RNA. Nucleic Acids Res. (1990), 18(2), 299-304. | |
| ²⁸. Chowrira, Bharat M.; Berzal-Herranz, Alfredo; Burke, John M.. Novel guanosine requirement for catalysis by the hairpin ribozyme. Nature (London) (1991), 354(6351), 320-2. | |
| ²⁹. Berzal-Herranz, Alfredo; Joseph, Simpson; Chowrira, Bharat M.; Butcher, Samuel E.; Burke, John M.. Essential nucleotide sequences and secondary structure elements of the hairpin ribozyme. EMBO J. (1993) 12(6), 2567-73. | |
| ³⁰. Joseph, Simpson; Berzal-Herranz, Alfredo; Chowrira, Bharat M.; Butcher, Samuel E.. Substrate selection rules for the hairpin ribozyme determined by in vitro selection, mutation, and analysis of mismatched substrates. Genes Dev. (1993), 7(1),130-8. | |
| ³¹. Berzal-Herranz, Alfredo; Joseph, Simpson; Burke, John M.. In vitro selection of active hairpin ribozymes by sequential RNA-catalyzed cleavage and litigation reactions. Genes Dev. (1992), 6(1), 129-34. | |
| ³². Hegg, Lisa A.; Fedor, Martha J.. Kinetics and Thermodynamics of Intermolecular Catalysis by Hairpin Ribozymes. Biochemistry (1995), 34(48), 15813-28. | |
| ³³. Grasby, Jane A.; Mersmann, Karin; Singh, Mohinder; Gait, Michael J.. Purine Functional Groups in Essential Residues of the Hairpin Ribozyme Required for Catalytic Cleavage of RNA. Biochemistry (1995), 34(48), 15813-28. | |
| ³³. Grasby, Jane A.; Mersmann, Karin; Singh, Mohinder; Gait, Michael J.. Purine Functional Groups in Essential Residues of the Hairpin Ribozyme Required for Catalytic Cleavage of RNA. Biochemistry (1995), 34(12), 4068-76. | |
| ³⁴. Schmidt, Sabine; Beigelman, Leonid; Karpeisky, Alexander; Usman, Nassim; Sorensen, Ulrik S.; Gait, Michael J.. Base and sugar requirements for RNA cleavage of essential nucleoside residues in internal loop B of the hairpin ribozyme: implications for secondary structure. Nucleic Acids Res. (1996), 24(4), 573-81. | |
| ³⁵. Perrotta, Anne T.; Been, Michael D.. Cleavage of oligoribonucleotides by a ribozyme derived from the hepatitis delta virus RNA sequence. Biochemistry (1992), 31(1), 16-21. | |
| ³⁶. Perotta, Anne T.; Been, Michael D.. A pseudoknot-like structure required for efficient self-deavage of hepatitis delta virus RNA. Nature (London) (1991), 350(6317), 434-6. | |
| ³⁷. Puttaraju, M.; Perrotta, Anne T.; Been, Michael D.. A circular transacting hepatitis delta virus ribozyme. Nucleic Acids Res. (1993), 21(18), 4253-8. | |

**TABLE 2**

| **2.5 µmol RNA Synthesis Cycle** | | | |
|---|---|---|---|
| **Reagent** | **Equivalents** | **Amount** | **Wait** |
| | | | **Time*** |
| Phosphoramidites | 6.5 | 163 µL | 2.5 |
| S-Ethyl Tetrazole | 23.8 | 238 µL | 2.5 |
| Acetic Anhydride | 100 | 233 µL | 5 sec |
| *N*-Methyl Imidazole | 186 | 233 µL | 5 sec |
| TCA | 83.2 | 1.73 mL | 21 sec |
| Iodine | 8.0 | 1.18 mL | 45 sec |
| Acetonitrile | NA | 6.67 mL | NA |

| | | | |
|---|---|---|---|
| * Wait time does not include contact time during delivery | | | |

Other embodiments are within the following claims.

## Claims

1. A nucleoside or a nucleotide comprising a nucleic acid sugar portion, wherein the 2' position of said sugar has the formula: 2'-O-NHR1, wherein R1 is selected from a group consisting of H, aminoacyl group, peptidyl group, biotinyl group, cholesteryl group, lipoic acid residue, retinoic acid residue, folic acid residue, ascorbic acid residue, nicotinic acid residue, 6-aminopenicillanic acid residue, 7-aminocephalosporanic acid residue, alkyl, alkenyl, alkynyl, aryl, alkylaryl, carbocyclic aryl, heterocyclic aryl, amide and ester.

2. A nucleoside or a nucleotide comprising a nucleic acid sugar portion, wherein the 2' position of said sugar has the formula: 2'-O-N=R3, wherein R3 is selected from a group consisting of pyridoxal residue, pyridoxal-5-phosphate residue, 13-cis-retinal residue, 9-cis-retinal residue, alkyl, alkenyl, alkynyl, alkylaryl, carbocyclic alkylaryl, and heterocyclic alkylaryl.

3. The nucleoside or nucleotide of claim 1 having the formula I: 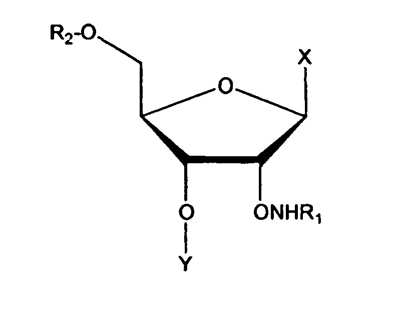 wherein R₁ is selected from a group consisting of H, aminoacyl group, peptidyl group, biotinyl group, cholesteryl group, lipoic acid residue, retinoic acid residue, folic acid residue, ascorbic acid residue, nicotinic acid residue, 6-aminopenicillanic acid residue, 7-aminocephalosporanic acid residue, alkyl, alkenyl, alkynyl, aryl, alkylaryl, carbocyclic aryl, heterocyclic aryl, amide and ester;
X is independently a nucleotide base or its analog;
Y is independently a phosphorus-containing group; and
R₂ is independently a blocking group or a phosphorus containing group.

4. The nucleoside or nucleotide of claim 2 having the formula III: 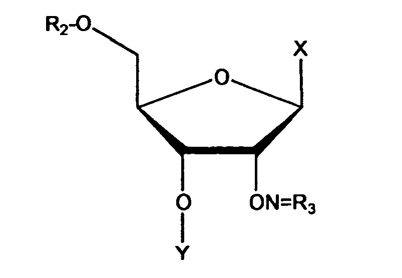 wherein R₃ is selected from a group consisting of pyridoxal residue, pyridoxal-5-phosphateresidue,13-cis-retinal residue, 9-cisretinal residue, alkyl, alkenyl, alkynyl, alkylaryl, carbocyclic alkylaryl, and heterocyclic alkylaryl;
X is independently a nucleotide base or its analog;
Y is independently a phosphorus-containing group; and
R₂ is independently a blocking group or a phosphorus-containing group.

5. A nucleoside or a nucleotide according to any of claims 1-4 comprising a nucleic acid base portion, wherein said base is selected from a group consisting of 2-fluoropyridine-3-yl, pyridine-2-one-3-yl, pyridin-2-(4-nitrophenylethyl)-one-3-yl, 2-bromopyridine-5-yl, pyridin-2-one-5-yl, 2-aminopyridine-5-yl, and pyridin-2-(4-nitrophenylethyl)-one-5-yl.

6. The nucleoside or nucleotide of claim 1 having the formula II: 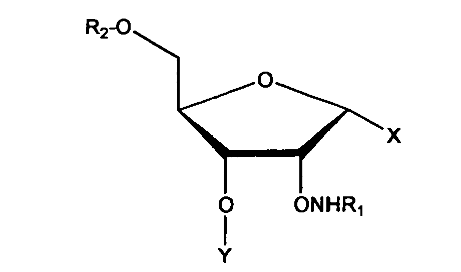 wherein R₁ is defined as in claim 1;
X is independently a nucleotide base selected from the group consisting of 2-fluoropyridine-3-yl, 2-bromopyridine-5-yl, pyridin-2-one-5-yl, and 2-aminopyridine-5-yl;
Y is independently a phosphorus-containing group; and
R₂ is independently a blocking group or a phosphorus-containing group.

7. The nucleoside or nucleotide of claim 1, 2, or 5, wherein said nucleoside or nucleotide is a nucleotide.

8. The nucleoside or nucleotide of claim 1, 2, or 5, wherein said nucleoside or nucleotide is a nucleotide-tri-phosphate.

9. A polynucleotide comprising the nucleoside or nucleotide of claim 1, 2, or 5 at one or more positions.

10. The polynucleotide of claim 9, wherein said polynucleotide is an enzymatic nucleic acid.

11. The enzymatic nucleic acid of claim 10, wherein said nucleic acid is in a hammerhead configuration.

12. The enzymatic nucleic acid of claim 10, wherein said nucleic acid is in a hairpin configuration.

13. The enzymatic nucleic acid of claim 10, wherein said nucleic acid is in a hepatitis delta virus, group I intron, VS RNA, group II intron or RNase P RNA configuration.

14. The nucleoside or nucleotide of claim 5, wherein said base is 2-fluoropyridin-3-yl.

15. The nucleoside or nucleotide of claim 5, wherein said base is pyridin-2-one-3-yl.

16. The nucleoside or nucleotide of claim 5, wherein said base is pyridin-2-(4-nitrophenylethyl)-one-3-yl.

17. The nucleoside or nucleotide of claim 6, wherein X is 2-fluoropyridin-3-yl.

18. The nucleoside or nucleotide of claim 5, wherein said base is 2-bromopyridin-5-yl.

19. The nucleoside or nucleotide of claim 6, wherein X is 2-bromopyridin-5-yl.

20. The nucleoside or nucleotide of claim 5, wherein said base is pyridin-2-one-5-yl.

21. The nucleoside or nucleotide of claim 6, wherein X is pyridin-2-one-5-yl.

22. The nucleoside or nucleotide of claim 5, wherein said base is 2-aminopyridin-5-yl.

23. The nucleoside or nucleotide of claim 5, wherein said base is pyridin-2-(4-nitrophenylethyl)-one-5-yl.

24. The nucleoside or nucleotide of claim 7, wherein X is 2-aminopyridin-5-yl.

25. The nucleoside or nucleotide of claim 1, wherein said nucleoside or nucleotide is 2'-O-amino adenosine.

26. The nucleoside or nucleotide of claim 1, wherein said nucleoside or nucleotide is 2'-O-amino guanosine.

27. The nucleoside or nucleotide of claim 1, wherein said nucleoside or nucleotide is 2'-O-amino cytidine.

28. The nucleoside or nucleotide of claim 1, wherein said nucleoside or nucleotide is 2'-O-amino uridine.

29. An isolated mammalian cell comprising the nucleoside or nucleotide of any one of the claims 1-6.

30. The isolated mammalian cell of claim 29, wherein said mammalian cell is a human cell.

31. An isolated mammalian cell comprising the polynucleotide of claim 9.

32. The isolated mammalian cell of claim 31, wherein said mammalian cell is a human cell.

33. A method of making a polynucleotide of claim 9.

34. A method of modulating gene expression using a polynucleotide of claim 9.

35. A pharmaceutical composition comprising a nucleoside or nucleotide of any of claims 1-6.

36. A pharmaceutical composition comprising a polynucleotide of claim 9.

37. The nucleoside or nucleotide of claim 1, 2, or 5 wherein said nucleoside or nucleotide is used as an antiviral agent.

38. A process for the synthesis of a 2'-O-amino nucleoside comprising the steps of:
contacting a 3' and 5'-protected arabino nucleoside with a sulfonylating reagent under conditions suitable for the formation of a 3' and 5'-protected 2'-arabino sulfonyl nucleoside;
displacement of the sulfonyl group from said 2'-arabino sulfonyl nucleoside with N-hydroxy-phthalimide in the presence of a strong organic base under conditions suitable for the formation of 3' and 5'-protected 2'-O-N-phthaloyl ribonucleoside;
deprotection of said phthaloyl ribonucleoside with a fluoride containing reagent under conditions suitable for the formation of 2'-O-N-phthaloyl ribonucleoside; and
contacting said 2'-O-N-phthaloyl ribonucleoside with a reagent selected from a group consisting of alkylamine, hydrazine, N-phenyl hydrazine and N-alkylhydrazine, under conditions suitable for the formation of said 2'-O-amino nucleoside.

39. The process of claim 38, wherein said sulfonylating reagent is tri-fluoromethane sulfonic anhydride.

40. The process of claim 38, wherein said sulfonylating reagent is tri-fluoromethane sulfonic chloride.

41. The process of claim 38, wherein said strong organic base is 1,8-diazabicyclo(5.4.0)undec-7-ene.

42. The process of claim 38, wherein said fluoride containing reagent is selected from a group consisting of tetrabutylammonium fluoride and triethylamine trihydrofluoride.

43. The process of claim 38, wherein said alkylamine is aqueous methylamine.

44. The process of claim 38, wherein said N-alkylhydrazine is N-methylhydrazine.

## Patentansprüche

1. Ein Nukleosid oder ein Nukleotid umfassend ein Nukleinsäurezuckeranteil, wobei die 2' Position von besagtem Zucker die Formel: 2'-O-NHR1 besitzt, wobei R1 ausgewählt wird aus einer Gruppe bestehend aus H, einer Aminoacylgruppe, einer Peptidylgruppe, einer Biotinylgruppe, einer Cholesterylgruppe, einem Liponsäurerest, einem Retinsäurerest, einem Folsäurerest, einem Ascorbinsäurerest, einem Nikotinsäurerest, einem 6-Aminopenicillansäurerest, einem 7-Aminocephalosporansäurerest, einem Alkyl, einem Alkenyl, einem Alkinyl, einem Aryl, einem Alkylaryl, einem carbozyklischen Aryl, einem heterozyklischen Aryl, einem Amid und einem Ester.

2. Ein Nukleosid oder ein Nukleotid umfassend einen Nukleinsäurezuckeranteil, wobei die 2'-Position von besagtem Zucker die Formel: 2'-O-N=R3 besitzt, wobei R3 ausgewählt wird aus einer Gruppe bestehend aus einem Pyridoxalrest, einem Pyridoxal-5-Phosphatrest, einem 13-cis-Retinalrest, einem 9-cis-Retinalrest, einem Alkyl, einem Alkenyl, einem Alkinyl, einem Alkylaryl, einem carbozyklischen Alkylaryl und einem heterozyklischen Alkylaryl.

3. Das Nukleosid oder Nukleotid gemäß Anspruch 1, das die Formel I besitzt: 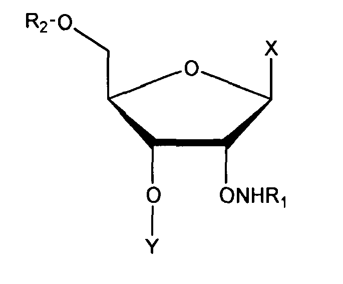 wobei R₁ ausgewählt wird aus einer Gruppe bestehend aus H, einer Aminoacylgruppe, einer Peptidylgruppe, einer Biotinylgruppe, einer Cholesterylgruppe, einem Liponsäurerest, einem Retinsäurerest, einem Folsäurerest, einem Ascorbinsäurerest, einem Nikotinsäurerest, einem 6-Aminopenicillansäurerest, einem 7-Aminocephalosporansäurerest, einem Alkyl, einem Alkenyl, einem Alkinyl, einem Aryl, einem Alkylaryl, einem carbozyklischen Aryl, einem heterozyklischen Aryl, einem Amid und einem Ester;
X unabhängig eine Nukleotidbase oder ein Analog davon ist;
Y unabhängig eine Phosphor-enthaltende Gruppe ist; und
R₂ unabhängig eine Blockier-Gruppe oder eine Phosphor-enthaltende Gruppe ist.

4. Das Nukleosid oder Nukleotid gemäß Anspruch 2, das die Formel III besitzt: 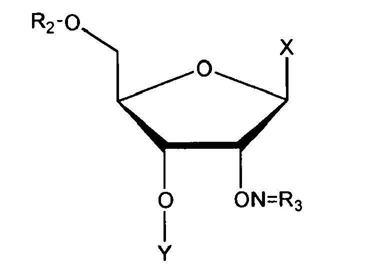 wobei R₃ ausgewählt wird aus einer Gruppe bestehend aus einem Pyridoxalrest, einem Pyridoxal-5-Phosphatrest, einem 13-cis-Retinalrest, einem 9-cis-Retinalrest, einem Alkyl, einem Alkenyl, einem Alkinyl, einem Alkylaryl, einem carbozyklischen Aryl und einem heterozyklischen Aryl;
X unabhängig eine Nukleotidbase oder ein Analog davon ist;
Y unabhängig eine Phosphor-enthaltende Gruppe ist; und
R₂ unabhängig eine Blockier-Gruppe oder eine Phosphor-enthaltende Gruppe ist.

5. Ein Nukleosid oder Nukleotid gemäß einem der Ansprüche 1-4 umfassend einen Nukleinsäurebasenanteil, wobei besagte Base ausgewählt wird aus einer Gruppe bestehend aus 2-Fluorpyridin-3-yl, Pyridin-2-on-3-yl, Pyridin-2-(4-Nitrophenylethyl)-on-3-yl, 2-Brompyridin-5-yl, Pyridin-2-on-5-yl, 2-Aminopyridin-5-yl und Pyridin-2-(4-Nitrophenylethyl)-on-5-yl.

6. Das Nukleosid oder Nukleotid gemäß Anspruch 1, das die Formel II besitzt: 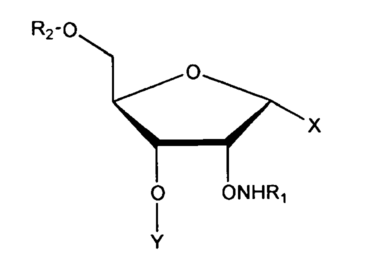 wobei R₁ wie in Anspruch 1 definiert ist;
X unabhängig eine Nukleotidbase ausgewählt aus der Gruppe bestehend aus 2-Fluorpyridin-3-yl, 2-Brompyridin-5-yl, Pyridin-2-on-5-yl und 2-Aminopyridin-5-yl ist;
Y unabhängig eine Phosphor-enthaltende Gruppe ist; und
R₂ unabhängig eine Blockier-Gruppe oder eine Phosphor-enthaltende Gruppe ist.

7. Das Nukleosid oder Nukleotid gemäß Anspruch 1, 2 oder 5, wobei besagtes Nukleosid oder Nukleotid ein Nukleotid ist.

8. Das Nukleosid oder Nukleotid gemäß Anspruch 1, 2 oder 5, wobei besagtes Nukleosid oder Nukleotid ein Nukleotid-Triphosphat ist.

9. Ein Polynukleotid umfassend das Nukleosid oder Nukleotid gemäß Anspruch 1, 2 oder 5 an einer oder mehreren Positionen.

10. Das Polynukleotid gemäß Anspruch 9, wobei besagtes Polynukleotid eine enzymatische Nukleinsäure ist.

11. Die enzymatische Nukleinsäure gemäß Anspruch 10, wobei besagte Nukleinsäure in einer Hammerkopf-Konfiguration ist.

12. Die enzymatische Nukleinsäure gemäß Anspruch 10, wobei besagte Nukleinsäure in einer Haarnadel-Konfiguration ist.

13. Die enzymatische Nukleinsäure gemäß Anspruch 10, wobei besagte Nukleinsäure in einer Hepatitis Delta Virus, Gruppe I Intron, VS RNA, Gruppe II Intron oder RNase P RNA Konfiguration ist.

14. Das Nukleosid oder Nukleotid gemäß Anspruch 5, wobei besagte Base 2-Fluorpyridin-3-yl ist.

15. Das Nukleosid oder Nukleotid gemäß Anspruch 5, wobei besagte Base Pyridin-2-on-3-yl ist.

16. Das Nukleosid oder Nukleotid gemäß Anspruch 5, wobei besagte Base Pyridin-2-(4-Nitrophenylethyl)-on-3-yl ist.

17. Das Nukleosid oder Nukleotid gemäß Anspruch 6, wobei X 2-Fluorpyridin-3-yl ist.

18. Das Nukleosid oder Nukleotid gemäß Anspruch 5, wobei besagte Base 2-Brompyridin-5-yl ist.

19. Das Nukleosid oder Nukleotid gemäß Anspruch 6, wobei X 2-Brompyridin-5-yl ist.

20. Das Nukleosid oder Nukleotid gemäß Anspruch 5, wobei besagte Base Pyridin-2-on-5-yl ist.

21. Das Nukleosid oder Nukleotid gemäß Anspruch 6, wobei X Pyridin-2-on-5-yl ist.

22. Das Nukleosid oder Nukleotid gemäß Anspruch 5, wobei besagte Base 2-Aminopyridin-5-yl ist.

23. Das Nukleosid oder Nukleotid gemäß Anspruch 5, wobei besagte Base Pyridin-2-(4-Nitrophenylethyl)-on-5-yl ist.

24. Das Nukleosid oder Nukleotid gemäß Anspruch 7, wobei X 2-Aminopyridin-5-yl ist.

25. Das Nukleosid oder Nukleotid gemäß Anspruch 1, wobei besagtes Nukleosid oder Nukleotid 2'-O-Amino Adenosin ist.

26. Das Nukleosid oder Nukleotid gemäß Anspruch 1, wobei besagtes Nukleosid oder Nukleotid 2'-O-Amino Guanosin ist.

27. Das Nukleosid oder Nukleotid gemäß Anspruch 1, wobei besagtes Nukleosid oder Nukleotid 2'-O-Amino Cytidin ist.

28. Das Nukleosid oder Nukleotid gemäß Anspruch 1, wobei besagtes Nukleosid oder Nukleotid 2'-O-Amino Uridin ist.

29. Eine isolierte Säugetierzelle umfassend das Nukleosid oder Nukleotid gemäß einem der Ansprüche 1-6.

30. Die isolierte Säugetierzelle gemäß Anspruch 29, wobei besagte Säugetierzelle eine menschliche Zelle ist.

31. Eine isolierte Säugetierzelle umfassend das Polynukleotid gemäß Anspruch 9.

32. Die isolierte Säugetierzelle gemäß Anspruch 31, wobei besagte Säugetierzelle eine menschliche Zelle ist.

33. Ein Verfahren zur Herstellung eines Polynukleotids gemäß Anspruch 9.

34. Ein Verfahren zur Modulation der Genexpression unter Verwendung eines Polynukleotids gemäß Anspruch 9.

35. Eine pharmazeutische Zusammensetzung umfassend ein Nukleosid oder Nukleotid gemäß einem der Ansprüche 1-6.

36. Eine pharmazeutische Zusammensetzung umfassend ein Polynukleotid gemäß Anspruch 9.

37. Das Nukleosid oder Nukleotid gemäß Anspruch 1, 2 oder 5, wobei besagtes Nukleosid oder Nukleotid als antivirales Mittel verwendet wird.

38. Ein Verfahren für die Synthese eines 2'-O-Amino Nukleosids umfassend die Schritte:
Inkontaktbringen eines 3' und 5'-geschützten Arabino-Nukleosids mit einem Sulfonierungsreagenz unter Bedingungen, die für die Bildung eines 3' und 5'-geschützten 2-Arabino-Sulfonyl-Nukleosids geeignet sind;
Ersetzen der Sulfonylgruppe von besagtem 2'-Arabino-Sulfonyl-Nukleosid mit N-Hydroxy-Phthalimid in Gegenwart einer starken organischen Base unter Bedingungen, die für die Bildung eines 3' und 5'-geschützten 2'-O-N-Phtaloyl-Ribonukleosids geeignet sind;
Entschützen von besagtem Phthaloyl-Ribonukleosid mit einem Fluor-enthaltenden Reagenz unter Bedingungen, die für die Bildung eines 2'-O-N-Phataloyl-Ribonukleosids geeignet sind; und
Inkontaktbringen von besagtem 2'-O-N-Phthaloyl-Ribonukleosid mit einem Reagenz ausgewählt aus der Gruppe bestehend aus Alkylamin, Hydrazin, N-Phenylhydrazin und N-Alkylhydrazin unter Bedingungen, die für die Bildung von besagtem 2'-O-Aminonukleosid geeignet sind.

39. Das Verfahren gemäß Anspruch 38, wobei besagtes Sulfonierungsreagenz Trifluormethansulfonsäureanhydrid ist.

40. Das Verfahren gemäß Anspruch 38, wobei besagtes Sulfonierungsreagenz Trifluormethansulfonsäurechlorid ist.

41. Das Verfahren gemäß Anspruch 38, wobei besagte starke organische Base 1,8-Diazabicyclo(5.4.0)undek-7-en ist.

42. Das Verfahren gemäß Anspruch 38, wobei besagtes Fluor-enthaltendes Reagenz ausgewählt wird aus der Gruppe bestehend aus Tetrabutylammoniumfluorid und Triethylamintrihydrofluorid.

43. Das Verfahren gemäß Anspruch 38, wobei besagtes Alkylamin wässriges Methylamin ist.

44. Das Verfahren gemäß Anspruch 38, wobei besagtes N-Alkylhydrazin N-Methylhydrazin ist.

## Revendications

1. Nucléoside ou nucléotide comprenant une partie sucre d'un acide nucléique, dans lequel la position 2' dudit sucre est de formule : 2'-O-NHR1, dans laquelle R1 est choisi dans un groupe constitué par H, un groupe aminoacyle, un groupe peptidyle, un groupe biotinyle, un groupe cholestéryle, un résidu acide lipoïque, un résidu acide rétinoïque, un résidu acide folique, un résidu acide ascorbique, un résidu acide nicotinique, un résidu acide 6-aminopénicillanique, un résidu acide 7-aminocéphalosporanique, un groupe alkyle, alcényle, alcynyle, aryle, alkylaryle, aryle carbocyclique, aryle hétérocyclique, un amide et un ester.

2. Nucléoside ou nucléotide comprenant une partie sucre d'un acide nucléique, dans lequel la position 2' dudit sucre est de formule : 2'-O-N=R3, dans laquelle R3 est choisi dans un groupe constitué par un résidu pyridoxal, un résidu pyridoxal-5-phosphate, un résidu 13-cis-rétinal, un résidu 9-cis-rétinal, un groupe alkyle, alcényle, alcynyle, alkylaryle, alkylaryle carbocyclique, et alkylaryle hétérocyclique.

3. Nucléoside ou nucléotide selon la revendication 1, de formule I : 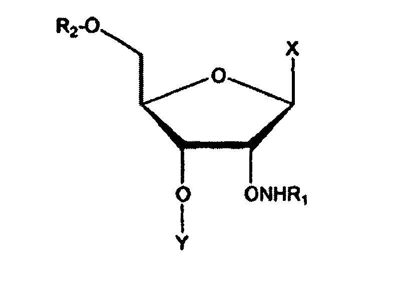 dans laquelle R₁ est choisi dans un groupe constitué par H, un groupe aminoacyle, un groupe peptidyle, un groupe biotinyle, un groupe cholestéryle, un résidu acide lipoïque, un résidu acide rétinoïque, un résidu acide folique, un résidu acide ascorbique, un résidu acide nicotinique, un résidu acide 6-aminopénicillanique, un résidu acide 7-aminocéphalosporanique, un groupe alkyle, alcényle, alcynyle, aryle, alkylaryle, aryle carbocyclique, aryle hétérocyclique, un amide et un ester ;
X est indépendamment une base nucléotidique ou son analogue ;
Y est indépendamment un groupe contenant du phosphore ; et
R₂ est indépendamment un groupe de blocage ou un groupe contenant du phosphore.

4. Nucléoside ou nucléotide selon la revendication 2, de formule III : 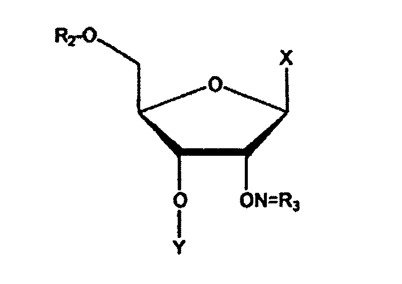 dans laquelle R₃ est choisi dans un groupe constitué par un résidu pyridoxal, un résidu pyridoxal-5-phosphate, un résidu 13-cisrétinal, un résidu 9-cisrétinal, un groupe alkyle, alcényle, alcynyle, alkylaryle, alkylaryle carbocyclique, et alkylaryle hétérocyclique ;
X est indépendamment une base nucléotidique ou son analogue ;
Y est indépendamment un groupe contenant du phosphore ; et
R₂ est indépendamment un groupe de blocage ou un groupe contenant du phosphore.

5. Nucléoside ou nucléotide selon l'une quelconque des revendications 1 à 4, comprenant une partie de base d'un acide nucléique, dans laquelle ladite base est choisie dans un groupe constitué par le 2-fluoropyridine-3-yl, le pyridine-2-one-3-yl, le pyridin-2-(4-nitrophényléthyl)-one-3-yl, le 2-bromopyridine-5-yl, le pyridin-2-one-5-yl, le 2-aminopyridine-5-yl, et le pyridin-2-(4-nitrophényléthyl)-one-5-yl.

6. Nucléoside ou nucléotide selon la revendication 1, de formule II : 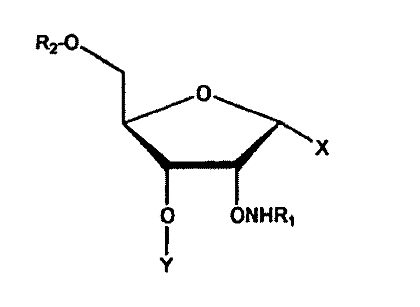 dans laquelle R₁ est tel que défini dans la revendication 1 ;
X est indépendamment une base nucléotidique choisie dans le groupe constitué par le 2-fluoropyridine-3-yl, le 2-bromopyridine-5-yl, le pyridin-2-one-5-yl, et le 2-aminopyridine-5-yl ;
Y est indépendamment un groupe contenant du phosphore ; et
R₂ est indépendamment un groupe de blocage ou un groupe contenant du phosphore.

7. Nucléoside ou nucléotide selon la revendication 1, 2, ou 5, dans lequel ledit nucléoside ou ledit nucléotide est un nucléotide.

8. Nucléoside ou nucléotide selon la revendication 1, 2, ou 5, dans lequel ledit nucléoside ou ledit nucléotide est un nucléotide-tri-phosphate.

9. Polynucléotide comprenant le nucléoside ou le nucléotide selon la revendication 1, 2, ou 5 à une ou plusieurs positions.

10. Polynucléotide selon la revendication 9, dans lequel ledit polynucléotide est un acide nucléique enzymatique.

11. Acide nucléique enzymatique selon la revendication 10, dans lequel ledit acide nucléique est dans une configuration en tête de marteau.

12. Acide nucléique enzymatique selon la revendication 10, dans lequel ledit acide nucléique est dans une configuration en épingle à cheveux.

13. Acide nucléique enzymatique selon la revendication 10, dans lequel ledit acide nucléique est dans une configuration du virus delta de l'hépatite, d'un intron du groupe I, d'ARN VS, d'un intron du groupe II ou d'ARN de la RNase P.

14. Nucléoside ou nucléotide selon la revendication 5, dans lequel ladite base est le 2-fluoropyridine-3-yl.

15. Nucléoside ou nucléotide selon la revendication 5, dans lequel ladite base est le pyridine-2-one-3-yl.

16. Nucléoside ou nucléotide selon la revendication 5, dans lequel ladite base est le pyridine-2-(4-nitrophényléthyl)-one-3-yl.

17. Nucléoside ou nucléotide selon la revendication 6, dans lequel X est le 2-fluoropyridine-3-yl.

18. Nucléoside ou nucléotide selon la revendication 5, dans lequel ladite base est le 2-bromopyridin-5-yl.

19. Nucléoside ou nucléotide selon la revendication 6, dans lequel X est le 2-bromopyridin-5-yl.

20. Nucléoside ou nucléotide selon la revendication 5, dans lequel ladite base est le pyridine-2-one-5-yl.

21. Nucléoside ou nucléotide selon la revendication 6, dans lequel X est le pyridin-2-one-5-yl.

22. Nucléoside ou nucléotide selon la revendication 5, dans lequel ladite base est le 2-aminopyridin-5-yl.

23. Nucléoside ou nucléotide selon la revendication 5, dans lequel ladite base est le pyridine-2-(4-nitrophényléthyl)-one-5-yl.

24. Nucléoside ou nucléotide selon la revendication 7, dans lequel X est le 2-aminopyridin-5-yl.

25. Nucléoside ou nucléotide selon la revendication 1, dans lequel ledit nucléoside ou ledit nucléotide est la 2'-O-amino adénosine.

26. Nucléoside ou nucléotide selon la revendication 1, dans lequel ledit nucléoside ou ledit nucléotide est la 2'-O-amino guanosine.

27. Nucléoside ou nucléotide selon la revendication 1, dans lequel ledit nucléoside ou ledit nucléotide est la 2'-O-amino cytidine.

28. Nucléoside ou nucléotide selon la revendication 1, dans lequel ledit nucléoside ou ledit nucléotide est la 2'-O-amino uridine.

29. Cellule de mammifère isolée comprenant le nucléoside ou le nucléotide selon l'une quelconque des revendications 1 à 6.

30. Cellule de mammifère isolée selon la revendication 29, dans laquelle ladite cellule de mammifère est une cellule humaine.

31. Cellule de mammifère isolée comprenant le polynucléotide selon la revendication 9.

32. Cellule de mammifère isolée selon la revendication 31, dans laquelle ladite cellule de mammifère est une cellule humaine.

33. Procédé de fabrication d'un polynucléotide selon la revendication 9.

34. Procédé de modulation de l'expression génique utilisant un polynucléotide selon la revendication 9.

35. Composition pharmaceutique comprenant un nucléoside ou un nucléotide selon l'une quelconque des revendications 1 à 6.

36. Composition pharmaceutique comprenant un polynucléotide selon la revendication 9.

37. Nucléoside ou nucléotide selon la revendication 1, 2, ou 5 dans lequel ledit nucléoside ou ledit nucléotide est utilisé en tant qu'un agent antiviral.

38. Procédé de synthèse d'un 2'-O-amino nucléoside comprenant les étapes consistant à :
mettre en contact un arabino nucléoside protégé en 3' et en 5' avec un réactif de sulfonylation dans des conditions adaptées pour la formation d'un 2'-arabino sulfonyl nucléoside protégé en 3' et en 5' ;
déplacer le groupe sulfonyle dudit 2'-arabino sulfonyl nucléoside avec du N-hydroxy-phtalimide en présence d'une base organique forte dans des conditions adaptées pour la formation d'un 2'-O-N-phtaloyl ribonucléoside protégé en 3' et en 5'; déprotéger ledit phtaloyl ribonucléoside avec un réactif contenant du fluorure dans des conditions adaptées pour la formation d'un 2'-O-N-phtaloyl ribonucléoside ; et
mettre en contact ledit 2'-O-N-phtaloyl ribonucléoside avec un réactif choisi dans un groupe constitué par une alkylamine, une hydrazine, une N-phényl hydrazine et une N-alkylhydrazine, dans des conditions adaptées pour la formation dudit 2'-O-amino nucléoside.

39. Procédé selon la revendication 38, dans lequel ledit réactif de sulfonylation est l'anhydride tri-fluorométhane sulfonique.

40. Procédé selon la revendication 38, dans lequel ledit réactif de sulfonylation est le chlorure tri-fluorométhane sulfonique.

41. Procédé selon la revendication 38, dans lequel ladite base organique forte est le 1,8-diazabicyclo(5.4.0)undéc-7-ène.

42. Procédé selon la revendication 38, dans lequel ledit réactif contenant du fluorure est choisi dans un groupe constitué par le fluorure de tétrabutylammonium et le trihydrofluorure de triéthylamine.

43. Procédé selon la revendication 38, dans lequel ladite alkylamine est la méthylamine aqueuse.

44. Procédé selon la revendication 38, dans lequel ladite N-alkylhydrazine est la N-méthylhydrazine.
